# EUROPÄISCHE PATENTSCHRIFT

(11) **EP 4 185 374 B1**
(45) Veröffentlichungstag und Bekanntmachung des Hinweises auf die Patenterteilung: **12.06.2024**
(21) Anmeldenummer: 21746723.2
(22) Anmeldetag: 20.07.2021
(51) Int. Cl.: A61N 1/40, A61N 1/32, A61N 1/04, A61L 2/03

(54) **VORRICHTUNG ZUR UNTERSTÜTZUNG EINER BEHANDLUNG MIT GEPULSTEN ELEKTRISCHEN FELDERN ZUR WUNDHEILUNG UND/ODER ZUR INAKTIVIERUNG VON MIKROORGANISMEN UND VERFAHREN ZUR INAKTIVIERUNG VON MIKROORGANISMEN**
DEVICE FOR SUPPORTING TREATMENT WITH PULSED ELECTRIC FIELDS FOR WOUND HEALING AND / OR FOR INACTIVATING MICROORGANISMS AND METHOD FOR INACTIVATING MICROORGANISMS
DISPOSITIF D'AIDE AU TRAITEMENT POURVU DE CHAMPS ÉLECTRIQUES PULSÉS DESTINÉ À LA GUÉRISON DES PLAIES ET/OU À L'INACTIVATION DES MICRO-ORGANISMES ET PROCÉDÉ D'INACTIVATION DES MICRO-ORGANISMES

(30) Priorität: 24.07.2020 EP 20187657
(43) Veröffentlichungstag der Anmeldung: 31.05.2023
(73) Patentinhaber: Hinterkopf-Theisen-Mogg Gesellschaft des bürgerlichen Rechts, 56075 Koblenz (DE)
(72) Erfinder: HINTERKOPF, Werner Gerhard, 56130 Bad Ems (DE); SRB, Josef, 33801 Blatna (CZ); KOROUS, Josef, 33801 Blatna (CZ); HINTERKOPF, Jan, 52070 Aachen (DE)
(74) Vertreter: Jacobi, Markus Alexander
(86) Internationale Anmeldenummer: PCT/EP2021/070244
(87) Internationale Veröffentlichungsnummer: WO 2022/018074

(56) Entgegenhaltungen:
- WO-A2-2006/116252
- CA-A1- 2 594 284
- GB-A- 326 534
- US-A1- 2013 068 226

## Beschreibung

Die Erfindung betrifft eine Vorrichtung zur Unterstützung der Wundheilung und/oder zur Inaktivierung von Mikroorganismen umfassend einen elektrischen Energiespeicher, einen Transformator zum Bereitstellen hochfrequenter elektrischer Pulse und ein Behandlungsinstrument, welches einen Körper aus einem elektrisch isolierenden Material und eine im Inneren des Körpers angeordnete Elektrode umfasst, wobei im Inneren des Körpers ein Gas oder Gasgemisch aufgenommen ist und das Behandlungsinstrument für eine Gasentladung bei elektrischer Anregung eingerichtet ist. Des Weiteren betrifft die Erfindung ein Verfahren zur Inaktivierung von Mikroorganismen, wobei eine solche Vorrichtung bereitgestellt wird und das Behandlungsinstrument der Vorrichtung mit hochfrequenten elektrischen Pulsen angeregt wird.

### Stand der Technik

In der Medizin und in anderen technischen Bereichen ist es notwendig Objekte wie medizinische Vorrichtungen oder biologisches Material wie Lebensmittel oder Gewebeproben zu desinfizieren oder zu sterilisieren. Typischerweise wird für eine Sterilisation gefordert, dass die Anzahl der lebenden Mikroorganismen um sechs Zehnerpotenzen vermindert wird. Für eine Desinfektion wird typischerweise eine Reduktion um fünf Zehnerpotenzen gefordert.

Auch bei der Behandlung von Menschen oder Tieren kann es notwendig sein, Gewebe wie eine Hautoberfläche oder eine offene Wunde zu behandeln, insbesondere zu desinfizieren, um Infektionen zu vermeiden und die Wundheilung zu fördern. Zur Desinfektion und zur Sterilisation sind verschiedene Methoden bekannt. Sofern ein zu sterilisierendes Objekt gegen erhöhte Temperaturen und Feuchtigkeit beständig ist, kann eine Sterilisierung in einem Autoklaven erfolgen. In einem Autoklaven wird das Objekt heißem Wasserdampf bei einer Temperatur von beispielsweise 131 °C ausgesetzt.

Je nach Beschaffenheit der zu desinfizierenden Objekte oder Materialien ist es jedoch nicht möglich diese zu autoklavieren. Biologisches Material wird durch die Wärmeeinwirkung zerstört und auch verschiedene in medizinischen Geräten eingesetzte Materialien sind nicht ausreichend beständig gegen die im Autoklaven wirkenden Bedingungen.

Im Stand der Technik sind auch weitere Verfahren zur Desinfektion bekannt, welche bei der Desinfektion nur eine geringe Erwärmung bewirken.

US 4,695,472 beschreibt ein Konservierungsverfahren und eine entsprechende Vorrichtung zur Konservierung flüssiger Nährmittel, bei dem das Nährmittel Pulsen eines hohen elektrischen Feldes ausgesetzt ist, welches eine Feldstärke von 5 kV/cm bis 25 kV/cm und eine Pulsdauer von 1 µs bis 100 µs bei einer Wiederholrate zwischen 0,1 Hz und 100 Hz aufweist. Das elektrische Feld wird dabei zwischen zwei Elektroden aufgebaut, wobei das Nährmittel in eine Behandlungszone zwischen den beiden Elektroden geführt wird.

Aus WO 2014/094931 A1 ist eine Vorrichtung zur Behandlung von biologischem Gewebe mit einem Niederdruckplasma bekannt. Die Vorrichtung umfasst eine elektrische Energieversorgung, einen Transformator zur Erzeugung eines hochfrequenten elektrischen Feldes und eine Sonde, welche mit dem Transformator elektrisch koppelbar ist, und eine Steuereinrichtung zur Steuerung des Transformators. Ein Transformatorgehäuse ist bevorzugt als ein Handgriff ausgebildet, welcher eine ergonomische Handhabung der Vorrichtung erlaubt, wobei die Energieversorgung außerhalb des Transformatorgehäuses angeordnet ist. Die Sonde ist mit einem Edelgas oder einem Edelgasgemisch mit einem Druck von 500 Pa bis maximal 3000 Pa gefüllt. Durch Anregung der Sonde über vom Transformator erzeugte elektrisch Pulse mit einer Spannung von 1,8 kV bis 35 kV bei einer Frequenz von 10 kHz bis 50 kHz und einer Pulslänge von etwa 250 µs wird zwischen der Sonde und dem zu behandelnden biologischen Gewebe ein Niederdruckplasma erzeugt, wobei die Sonde dazu in einem Abstand von 1 mm bis 50 mm von dem Gewebe positioniert ist. Das biologische Gewebe bildet dabei eine Erdelektrode aus, welche mit einer Erdung der Spannungsquelle verbunden ist.

Die aus WO2014/094931 A1 bekannte Vorrichtung kann zur Behandlung von Gewebe von Menschen oder Tieren, insbesondere von Hautoberflächen und auch von offenen Wunden eingesetzt werden. Eine über das Desinfizieren des Gewebes hinausgehende Förderung der Wundheilung wird jedoch nicht erzielt.

WO2011/015538 beschreibt eine Vorrichtung zur Erzeugung eines nichtthermischen Atmosphärendruck-Plasmas. Die Vorrichtung umfasst ein im Bereich des austretenden Plasmas als geerdete Elektrode fungierendes Metallgehäuse, in dem ein HF-Generator, eine HF-Resonanzspule mit einem, für die Hochfrequenz geeigneten geschlossenen Ferritkern, ein als Gasdüse fungierender Isolierkörper sowie eine in dem Isolierkörper gehalterte Hochspannungs-Elektrode derart angeordnet sind, dass sie vom Prozessgas um- bzw. durchströmt werden. Zur Anregung wird eine Hochfrequenz mit ca. 1 MHz verwendet.

US 2013/068226 A1 beschreibt eine Vorrichtung zum Bereitstellen eines kalten Plasmas für die Inhalation. Ein biokompatibles Gas wird hierzu in eine Vorrichtung eingeleitet und einer dielektrischen Barriereentladung ausgesetzt. Die Vorrichtung weist eine Maske zum Aufsetzen für einen Patienten auf, wobei ein Modul zur Erzeugung der Plasmaentladung in eine Mundöffnung eingesetzt werden kann.

WO 2006/116252 beschreibt eine Vorrichtung zur Anwendung nicht-thermischer Plasmen an lebendem Gewebe. Hierzu soll in der Nähe des Gewebes ein Plasma erzeugt werden, ohne das Gewebe zu erhitzen. Zwischen der Vorrichtung und dem zu behandelnden Patienten ist dabei ein Abstand (plasma gap) einzuhalten, um das kalte Plasma zu erzeugen.

Nachteilig an den bekannten Vorrichtungen und Verfahren zur Behandlung von Gewebe mit Niederdruckplasma ist, dass eine präzise Einhaltung des Abstands zwischen dem Behandlungsinstrument bzw. der Sonde und dem Gewebe schwierig ist. Des Weiteren wirken die bekannten Verfahren lediglich oberflächlich, es wird keine Wirkung in der Tiefe des Gewebes erzielt. Bei den bekannten Vorrichtungen und Verfahren auf Basis von Pulsen hoher elektrischer Felder ist problematisch, dass zwei Elektroden benötigt werden, zwischen denen die Behandlungszone mit dem gepulsten hohen elektrischen Feld entsteht. Insbesondere wird eine Behandlung von großen Flächen eines Objekts dadurch erheblich erschwert.

Es ist somit eine Aufgabe der Erfindung eine Vorrichtung und ein Verfahren bereitzustellen, mit der bzw. mit dem eine Inaktivierung von Mikroorganismen auch in der Tiefe des Gewebes erzielt wird. Des Weiteren ist es eine Aufgabe der Erfindung eine Vorrichtung bereitzustellen, mit der die Wundheilung unterstützt werden kann. Insbesondere ist es eine Aufgabe, eine Stimulierung der natürlichen vier Phasen der Wundheilung vorzunehmen, um die Wundheilung zu unterstützen. Ferner ist es eine Aufgabe der Erfindung eine Vorrichtung bereitzustellen, welche keine Erdung oder zweite Elektrode für die Durchführung der Behandlung erfordert.

### Offenbarung der Erfindung

Die Erfindung ist in den unabhängigen Ansprüchen 1 und 12 definiert.

Es wird eine Vorrichtung zur Unterstützung der Wundheilung und/oder zur Inaktivierung von Mikroorganismen vorgeschlagen, welche einen elektrischen Energiespeicher, einen Pulsgenerator zur Bereitstellung elektrischer Anregungspulse und einen Transformator zum Bereitstellen hochfrequenter elektrischer Pulse an einer Ausgangsseite umfasst. Dabei ist ein erster Anschluss einer Eingangsseite des Transformators mit dem Pulsgenerator verbunden. Die Vorrichtung umfasst ferner ein Behandlungsinstrument, welches einen geschlossenen Körper aus einem elektrisch isolierenden Material und eine im Inneren des Körpers angeordnete Elektrode umfasst, wobei im Inneren des Körpers ein Gas oder Gasgemisch aufgenommen ist und das Behandlungsinstrument für eine Gasentladung bei elektrischer Anregung eingerichtet ist, wobei ein erstes Ende des Behandlungsinstrument für eine Kopplung mit einem ersten Anschluss der Ausgangsseite des Transformators eingerichtet ist.

Ferner ist vorgesehen, dass ein zweites Ende des Behandlungsinstruments zum Kontaktieren von Oberflächen und/oder von biologischem Gewebe eingerichtet ist und dass ein zweiter Anschluss der Ausgangsseite des Transformators mit einem zweiten Anschluss einer Eingangsseite des Transformators sowie einer Gehäuseabschirmung verbunden ist, wobei die Gehäuseabschirmung durch ein Gehäuse der Vorrichtung elektrisch von der Außenwelt isoliert ist und somit als erdfreie Masse eingerichtet ist und wobei der Transformator und der Pulsgenerator derart eingerichtet sind, dass die hochfrequenten elektrischen Pulse eine Frequenz im Bereich von 10 kHz bis 100 kHz aufweisen. Ferner weisen die hochfrequenten elektrischen Pulse bevorzugt eine Pulsbreite im Bereich von 1 µs bis 1000 µs auf. Eine Pulswiederholrate der hochfrequenten elektrischen Pulse liegt bevorzugt im Bereich von 100 Hz bis 400 Hz. Ein Puls-Pause Verhältnis liegt bevorzugt im Bereich von 0,01% bis 1%. Das Behandlungsinstrument ist dabei dazu eingerichtet, die erzeugten hochfrequenten elektrischen Pulse bzw. deren elektrische Felder zu einem zu behandelnden Objekt oder zu einem zu behandelnden organischen bzw. biologischen Material zu leiten.

Im Betrieb wird durch die elektrischen Pulse im Inneren des Behandlungsinstruments eine Gasentladung angeregt, wobei das Innere des geschlossenen Körpers mit einem geeigneten Gas gefüllt ist. In der Umgebung des Behandlungsinstruments tritt ein dielektrischer Verschiebungsstrom auf, wobei sich bei Kontakt des Behandlungsinstruments mit einer Oberfläche eines Objekts bzw. von organischem Material der dielektrische Verschiebungsstrom an der Stelle der Berührung konzentriert. Dabei ist vorgesehen, dass im Betrieb der Vorrichtung das Behandlungsinstrument bzw. der an der Oberfläche des Behandlungsinstruments vorliegende dielektrische Verschiebungsstrom die einzige elektrische Verbindung der Vorrichtung nach außen ist. Insbesondere ist das im geschlossenen Körper aufgenommene Gas von der Umgebung abgeschlossen, so dass die Gasentladung auf das Innere des Behandlungsinstruments beschränkt ist.

Die Vorrichtung weist insbesondere keine Erdung auf und kontaktiert das zu behandelnde Objekt ausschließlich über das Behandlungsinstrument. Weitere elektrische Verbindungen zwischen dem zu behandelnden Objekt und der Vorrichtung sind nicht vorgesehen und werden durch ein elektrisch isolierendes Gehäuse der Vorrichtung unterbunden.

Zur Erzeugung der hochfrequenten elektrischen Pulse umfasst die Vorrichtung einen Transformator, welcher elektrische Anregungspulse mit geringer Spannung in eine Hochspannung transformiert. Die Hochspannung wird an der Ausgangsseite des Transformators bereitgestellt, wobei die Ausgangsseite mit dem Behandlungsinstrument elektrisch gekoppelt ist. Die elektrischen Anregungspulse geringer Spannung werden an einer Eingangsseite des Transformators als Ansteuersignal zur Verfügung gestellt, beispielsweise durch einen Pulsgenerator oder eine Steuereinheit.

Bevorzugt ist der Transformator als ein impulsbetriebener Resonanztransformator mit einem Magnetkern ausgebildet. Bei einem solchen impulsbetriebenen Resonanztransformator sind eine Primärspule und eine Sekundärspule auf dem gemeinsamen Magnetkern angeordnet. Der Magnetkern ist dabei bevorzugt als ein Ferritkern ausgebildet. Für das Bereitstellen der hochfrequenten elektrischen Pulse wird der impulsbetriebene Resonanztransformator mit den Spannungspulsen angeregt, wobei eine Wiederholrate der elektrischen Anregungspulse der Pulswiederholrate der hochfrequenten elektrischen Pulse entspricht.

Bei dem impulsbetriebenen Resonanztransformator mit Magnetkern sind die Primärspule, welche die Eingangsseite des Transformators darstellt, und die Sekundärspule, welche die Ausgangsseite des Transformators darstellt, miteinander über den Magnetkern magnetisch gekoppelt. Die Sekundärspule weist dabei eine höhere Windungszahl auf, als die Primärspule. Die Primärspule weist beispielsweise im Bereich von 2 bis 100, bevorzugt im Bereich von 4 bis 40 Windungen auf. Die Sekundärspule weist beispielsweise im Bereich von 1000 bis 100000 Windungen auf.

Der Magnetkern ist bevorzugt im Wesentlichen Stabförmig ausgebildet, wobei sowohl die Primärspule als auch die Sekundärspule um den Magnetkern gewickelt sind. Die Länge des Magnetkerns beträgt bevorzugt 1 cm bis 6 cm. Der Magnetkern kann dabei Kammern ausbilden, um die einzelnen Windungen der Spulen aufzunehmen.

Bevorzugt ist die Sekundärspule in mehrere Wicklungsabschnitte unterteilt, welche jeweils voneinander beabstandet sind. Beispielsweise kann der Magnetkern, welche hier auch als ein Wickelkern dient, auf den die Sekundarspule und die Primärspule gewickelt sind, mehrere Spulenaufnahmen bereitstellen, wobei jeweils die Wicklungen in einer der Spulenaufnahmen einen Wicklungsabschnitt der Sekundärspule darstellen. Beispielsweise können 2 bis 20, bevorzugt 2 bis 15 Wicklungsabschnitte vorgesehen sein. Besonders bevorzugt sind 3 bis 9 Wicklungsabschnitte vorgesehen. Jeder Wicklungsabschnitt kann beispielsweise 100 bis 1000 Windungen der Sekundärspule aufweisen.

Auch die Primärspule kann in mehrere Abschnitte unterteilt werden. Beispielsweise kann die Primärspule in Form von 1 bis 5 Wicklungsabschnitten auf dem als gemeinsamen Wickelkern dienenden Magnetkern angeordnet werden, wobei die einzelnen Abschnitte der Primärspule räumlich voneinander getrennt werden, beispielsweise durch das Vorsehen von mehreren Spulenaufnahmen auf dem Wickelkern.

Besonders bevorzugt wird die Primärspule zwischen den Wicklungsabschnitten der Sekundärspule auf dem Magnetkern angeordnet. Dabei kann die Primärspule beispielsweise in Form eines einzigen Abschnitts ausgestaltet sein und somit vollständig zwischen zwei Wicklungsabschnitten der Sekundärspule angeordnet sein. Alternativ weist die Primärspule ebenfalls mehrere Wicklungsabschnitte auf, wobei jeweils ein Wicklungsabschnitt der Primärspule zwischen zwei Wicklungsabschnitten der Sekundärspule angeordnet ist.

Als Draht für das Wickeln der Spulen wird bevorzugt ein Kupferdraht eingesetzt, wobei der Durchmesser für den Kupferdraht der Sekundärspule bevorzugt im Bereich von 0,01 mm und 0,1 mm, besonders bevorzugt im Bereich von 0,03 mm bis 0,07 mm beträgt. Für die Primärspule wird bevorzugt ein dickerer Kupferdraht verwendet, der bevorzugt einen drei bis 10-fach höheren Durchmesser aufweist, als der für die Sekundärspule verwendete Kupferdraht.

Die Frequenz der erzeugten hochfrequenten elektrischen Pulse wird im Wesentlichen durch eine Resonanzfrequenz des aus der Sekundärspule des impulsbetriebenen Transformators und des Behandlungsinstruments gebildeten schwingfähigen Systems bestimmt. Insbesondere bestimmt sich die Resonanzfrequenz aus der Induktivität der Sekundärspule, eine Kapazität der Spule und der Kapazität des Behandlungsinstruments. Die Anregung dieses schwingfähigen Systems mit den elektrischen Anregungspulsen wird bevorzugt an diese Resonanzfrequenz angepasst. Eine Feineinstellung der Anregung des impulsbetriebenen Transformators erfolgt bevorzugt durch Verschieben der Lage der Primärspule in Bezug auf die Sekundärspule. Des Weiteren kann durch die Wahl der Abstände zwischen den einzelnen Wicklungsabschnitten und der Anzahl der Wicklungsabschnitte insbesondere eine elektrische Kapazität verändert werden, wodurch die Eigenfrequenz bzw. Resonanzfrequenz beeinflusst werden kann. Insbesondere ist es hierbei bevorzugt, die Abstände der Wicklungsabschnitte zwischen zwei benachbarten Abschnitten der Sekundärspule zu variieren.

Der impulsbetriebene Transformator sowie das Behandlungsinstrument sind derart ausgestaltet, dass die Eigenfrequenz bzw. Resonanzfrequenz des an der Ausgangsseite des Transformators gebildeten schwingfähigen Systems im Bereich von 10 kHz bis 100 kHz, bevorzugt im Bereich von 20 kHz bis 80 kHz und besonders bevorzugt im Bereich von 30 kHz bis 70 kHz liegt.

Dabei wird bevorzugt berücksichtigt, dass sich diese im Wesentlichen aus der Induktivität der Sekundärspule und der Kapazität des Behandlungsinstruments gegebene Resonanzfrequenz verschiebt, wenn das Behandlungsinstrument ein Objekt berührt. Durch das Berühren eines Objekts mit dem Behandlungsinstrument ändert sich die elektrische Kapazität des schwingfähigen Systems, was entsprechend zu einer Änderung der Frequenz führt.

Beispielsweise sind das Behandlungsinstrument und der Transformator derart eingerichtet, dass sich an der Ausgangsseite bei Anregung mit einem elektrischen Anregungspuls in Form eines Gleichspannungspulses von beispielsweise 25 V mit einer Dauer von beispielsweise 9 µs, ein hochfrequenter elektrischer Puls mit einer Frequenz von 60 kHz bis 70 kHz einstellt, wenn das Behandlungsinstrument nicht mit einem Objekt in Kontakt ist. Der erzeugte hochfrequente elektrische Puls hat direkt nach der Anregung mit dem elektrischen Anregungspuls seine höchste Amplitude und fällt dann aufgrund der gedämpften Schwingung ab. Die Pulsbreite des hochfrequenten elektrischen Pulses ist wesentlich Länger als die Dauer des elektrischen Anregungspulses.

Wird das Behandlungsinstrument beispielsweise von einer Person berührt, so ändert sich die elektrische Kapazität des schwingfähigen Systems an der Ausgangsseite des Transformators. Beispielsweise beträgt die Resonanzfrequenz des schwingfähigen Systems an der Ausgangsseite des impulsbetriebenen Transformators ca. 40 kHz.

Die Pulsbreite der elektrischen Anregungspulse, also die zeitliche Dauer eines elektrischen Anregungspulses, liegt bevorzugt im Bereich von 0,1 µs bis 20 µs, besonders bevorzugt im Bereich von 0,5 µs bis 15 µs und ganz besonders bevorzugt im Bereich von 1 µs bis 10 µs wird. Beispielsweise beträgt die Pulsdauer 9 µs.

Die Pulswiederholrate, welche bestimmt, wie viele elektrische Anregungspulse pro Sekunde erzeugt werden, liegt im Bereich von 100 Hz bis 400 Hz. Bevorzugt beträgt die Pulswiederholrate im Bereich von 200 Hz bis 300 Hz, beispielsweise beträgt die Pulswiederholrate 280 Hz. Die Pulswiederholrate der elektrischen Anregungspulse entspricht der Pulswiederholrate der hochfrequenten elektrischen Pulse.

Die Pulsbreite der hochfrequenten elektrischen Pulse, also die zeitliche Dauer eines hochfrequenten elektrischen Pulses, liegt im Bereich von 1 µs bis 1000 µs, bevorzugt im Bereich von 50 µs bis 500 µs und besonders bevorzugt im Bereich von 100 µs bis 250 µs wird. Die Pulsbreite wird insbesondere durch eine Dämpfung der vom elektrischen Anregungspuls angeregten Schwingung des gebildeten schwingfähigen Systems aus Transformator und Behandlungsinstrument bestimmt.

Aus der gewählten Pulsbreite bzw. Pulsdauer und der Pulswiederholrate der hochfrequenten elektrischen Pulse bestimmt sich das Puls-Pause-Verhältnis. Dieses Puls-Pause-Verhältnis, auch als duty-cycle oder Tastverhältnis bezeichnet, gibt das Verhältnis zwischen der Pulsbreite und der sich bis zum Ausgeben des nächsten elektrischen Pulses anschließenden Pause an. Das Puls-Pause-Verhältnis liegt im Bereich von 0,01 % bis 1 %, bevorzugt im Bereich von 0,05% bis 0,5% und besonders bevorzugt im Bereich von 0,1% bis 0,25%. Bei einer Pulsdauer von beispielsweise 9 µs und einer Wiederholrate von beispielsweise 250 Hz beträgt das Puls-Pause-Verhältnis 0,225%.

Bevorzugt ist eine Steuereinheit vorgesehen, welche den Transformator über den Pulsgenerator mit elektrischen Anregungspulsen geringer Spannung bzw. dem Ansteuersignal ansteuert. Die Spannung der elektrischen Anregungspulse liegt beispielsweise im Bereich von 10 V bis 70 V und wird vom Transformator in die Hochspannung bzw. die hochfrequenten elektrischen Pulse transformiert. Besonders bevorzugt liegt die Spannung im Bereich von 15 V bis 40 V. Die Wiederholrate der elektrischen Anregungspulse entspricht dabei der Pulswiederholrate der hochfrequenten elektrischen Pulse. Die Pulsdauer der elektrischen Anregungspulse hingegen ist wesentlich geringer als die Pulsbreite der hochfrequenten elektrischen Pulse. Insbesondere ist die Dauer der elektrischen Anregungspulse bevorzugt mindestens um den Faktor 5, besonders bevorzugt mindestens um den Faktor 10 kürzer als die Pulsbreite des hochfrequenten elektrischen Pulses.

Die Hochspannung der hochfrequenten elektrischen Pulse liegt bevorzugt im Bereich von 5 kV bis 40 kV. Besonders bevorzugt liegt die Hochspannung im Bereich von 10 bis 25 kV. Diese Angaben beziehen sich dabei auf die maximale Peak-Peak Spannung der hochfrequenten elektrischen Pulse.

Bevorzugt ist die Vorrichtung eingerichtet, einen Strom an der Ausgangsseite des Transformators auf einen Maximalwert im Bereich von 1 µA bis 300 µA zu begrenzen. Besonders bevorzugt wird der Strom an der Ausgangsseite auf einen Maximalwert im Bereich von 10 µA bis 120 µA begrenzt. Beispielsweise wird der maximale Strom auf 100 µA begrenzt. Für eine Begrenzung des maximalen Stroms können Mittel wie beispielsweise ein elektrischer Widerstand, eine Sicherung oder eine Überwachungs- oder Sicherheitseinrichtung eingesetzt werden, welche beispielsweise den Strom direkt oder indirekt misst und bei Überschreiten eines Grenzwerts die Stromzufuhr unterbricht.

Durch die vorgeschlagenen Parameter für die hochfrequenten elektrischen Pulse, welche an der Ausgangsseite des Transformators erzeugt und an das mit der Ausgangsseite des Transformators gekoppelte Behandlungsinstrument weitergebenen werden, wird eine Begrenzung der abgegebenen Leistung erreicht. Es ist somit ausreichend, die Einhaltung dieser Parameter, also insbesondere der Spannung und Frequenz der hochfrequenten elektrischen Pulse, zu überwachen.

Durch eine Begrenzung der abgegebenen Leistung der Vorrichtung wird gewährleistet, dass eine übermäßige Erwärmung von Objekten oder biologischem Material, welches mit der Vorrichtung behandelt wird, vermieden wird. Insbesondere bei Anwendung an einem Menschen oder an einem Tier ist eine Erwärmung derart zu beschränken, dass sich die Haut innerhalb von 10 Minuten maximal um 3°C erwärmt. Bei Anwendung am Menschen darf ferner keine Erwärmung über eine Temperatur von 41°C hinaus erfolgen.

Bevorzugt umfasst die Vorrichtung eine Sicherheitseinrichtung, welche eingerichtet ist, die elektrischen Parameter an der Ausgangsseite des Transformators zu überwachen und bei Überschreitung bzw. Unterschreitung vorgegebener Grenzwerte für einen der elektrischen Parameter eine Energiezufuhr zum Transformator zu unterbrechen.

Die überwachten elektrischen Parameter an der Ausgangsseite sind bevorzugt ausgewählt aus dem elektrischen Strom, der Spannung, der Pulswiederholrate, der Pulsbreite, der Pulsfrequenz und Kombinationen mindestens zweier dieser Parameter. Beispielsweise werden Spannung und Frequenz überwacht. Bevorzugt werden alle der genannten elektrischen Parameter überwacht.

Bevorzugt werden für die überwachten elektrischen Parameter Grenzwerte definiert, wobei bei einer Überschreitung eines oberen Grenzwerts bzw. bei Unterschreitung eines unteren Grenzwerts die Energiezufuhr zum Transformator unterbrochen wird. Die Grenzwerte können beispielsweise als eine prozentuale Abweichung vorgegeben werden. Die dabei zugelassene Abweichung beträgt bevorzugt maximal 5% und besonders bevorzugt maximal 3%. Beispielsweise kann bei einer Sollwiederholrate von 280 Hz eine Abweichung von +/- 5 % vorgegeben werden, so dass bei einer Wiederholrate von mehr als 294 Hz oder weniger als 266 Hz eine Abschaltung erfolgt.

Die Sicherheitseinrichtung umfasst bevorzugt entsprechende Sensoren, um die jeweiligen elektrischen Parameter zu erfassen. Insbesondere kann dabei eine zeitaufgelöste Messung der Spannung erfolgen, wobei durch digitale Verarbeitung daraus weitere elektrische Parameter wie (peak) Spannung, Frequenz, Pulsbreite bzw. Pulslänge, Puls-Pause-Verhältnis und Pulswiederholrate bestimmt werden. Des Weiteren kann ein Stromsensor vorgesehen sein, um den Stromfluss zu messen.

Mittel zur Messung der Spannung an der Primärspule oder an der Sekundärspule sind bevorzugt hochohmig ausgeführt, um die Spannungsquelle, welche ebenfalls hochohmig ist, möglichst gering zu belasten. Die Mittel können insbesondere einen Analog/Digital-Wandler umfassen, um die Spannung zu messen. Eine Auswertung des Messergebnisses und insbesondere ein Vergleich mit vorgegebenen Grenzwerten kann dann beispielsweise über ein Computerprogramm erfolgen, welches durch einen geeigneten Prozessor wie z.B. einem Mikrocontroller, ausgeführt wird.

Eine Messung an der Sekundärseite des Transformators kann beispielsweise durch Messen einer Spannung an einem Kondensator erfolgen, der über eine Diode, einen Impedanzwandler und einen Spannungsteiler mit der Ausgangsseite des Transformators verbunden ist. Der Impedanzwandler kann beispielsweise als ein Operationsverstärker ausgestaltet sein und der Spannungsteiler kann über Widerstände oder über Kondensatoren realisiert werden.

Bevorzugt erfolgt eine Messung der Spannung und/oder der Frequenz des hochfrequenten elektrischen Pulses während einer Anregungspause des Resonanztransformators über eine Messung der an der Primärseite des Resonanztransformators induzierten Spannung. Auf diese Weise sind keine direkten Messungen an der Hochspannungsseite des Transformators erforderlich. Dabei kann beispielsweise direkt die an der Primärspule während der Anregungspause anliegende Spannung gemessen werden.

Eine besonders einfache Messung zur Kontrolle der Spannung kann beispielsweise realisiert werden, indem über eine Diode die an der Primärseite durch Schwingungen in dem Resonanztransformator induzierte Spannung verwendet wird, um einen Kondensator aufzuladen. Bevorzugt erfolgt dabei die Verbindung zur Primärspule über einen Impedanzwandler sowie gegebenenfalls über einen Spannungsteiler. Über einen steuerbaren Schalter, der beispielsweise als MOSFET ausgestaltet ist, kann während des elektrischen Anregungspulses der Ausgang des Impedanzwandlers mit der Gehäuseabschirmung und somit mit dem zweiten Anschluss der Primärspule kurzgeschlossen werden, so dass der Anregungspuls den Kondensator nicht aufladen kann. Erst nach Beenden der Anregung des Transformators wird der steuerbare Schalter sperrend geschaltet, so dass jeweils die positiven Halbwellen der im Transformator vorliegenden Schwingung den Kondensator aufladen. Die am Kondensator messbare Spannung ist repräsentativ für die Spannung an der Ausgangsseite, also an der Sekundärspule, des Transformators. Der Impedanzwandler kann beispielsweise als ein Operationsverstärker ausgebildet sein und der Spannungsteiler kann über Widerstände oder über Kondensatoren realisiert werden.

Zusätzlich oder alternativ umfasst die Vorrichtung bevorzugt eine Sicherheitseinrichtung, welche eingerichtet ist, eine elektrische Verbindung der Vorrichtung zu einem Stromnetz zu erkennen und bei Vorliegen einer solchen Verbindung eine Energiezufuhr zum Transformator zu unterbrechen. Hierdurch wird erreicht, dass während des Betriebs keine Verbindung zwischen der Vorrichtung und einem Stromnetz besteht und somit eine Gefährdung von Nutzern durch Netzspannung während des Betriebs der Vorrichtung ausgeschlossen ist.

Bevorzugt weist die Vorrichtung ein Gehäuse in Form eines Handgriffs auf, wobei in dem Gehäuse der elektrische Energiespeicher, der Transformator und ein Teil des Behandlungsinstruments untergebracht sind. Ein anderer Teil des Behandlungsinstruments ragt aus dem Gehäuse heraus. Besonders bevorzugt ist das Gehäuse derart ausgestaltet, dass bis auf den aus dem Gehäuse herausragenden Teil des Behandlungsinstruments sämtliche Komponenten der Vorrichtung im Gehäuse aufgenommen sind.

Das Gehäuse weist bevorzugt eine äußere Gehäuseschale auf, welche aus einem elektrisch isolierenden Material gefertigt ist. Das Material der Gehäuseschale ist insbesondere ein Kunststoff.

Das Gehäuse umfasst bevorzugt eine als Gehäuseabschirmung ausgebildete elektrische Abschirmung, welche an einer Innenseite der Gehäuseschale angeordnetes ist und elektrisch leitfähig ist. Bevorzugt ist die Abschirmung in Form einer elektrisch leitenden Folie und/oder in Form elektrisch leitfähiger Platten ausgestaltet oder in Form einer elektrisch leitfähigen Beschichtung der Innenseite der Gehäuseschale ausgeführt. Beispielsweise wird eine elektrisch leitfähige Folie, wie beispielsweise Aluminiumfolie, als Abschirmung verwendet.

Die elektrische Abschirmung umschließt die Vorrichtung bevorzugt vollständig, wobei lediglich für das aus dem Gehäuse herausragende Behandlungsinstrument und gegebenenfalls für Anschlüsse und/oder Bedienelemente Öffnungen in der Abschirmung vorgesehen sind.

Die Gehäuseabschirmung ist durch das Gehäuse vollständig isoliert, so dass keine leitfähige Verbindung von der Gehäuseabschirmung nach außen erfolgt. Insbesondere erfolgt keine leitfähige Verbindung zwischen der Gehäuseabschirmung mit einer Person, welche die Vorrichtung für eine Behandlung eines Objekts führt, und es erfolgt keine leitfähige Verbindung zwischen der Gehäuseabschirmung und dem zu behandelnden Objekt.

Bevorzugt umfasst die Vorrichtung einen Ladeanschluss, über den der elektrische Energiespeicher der Vorrichtung aufgeladen werden kann. Bevorzugt ist der elektrische Energiespeicher als ein Akkumulator ausgeführt, beispielsweise als ein Lithium-Ionen Akkumulator oder als ein Nickel-Metallhydrid Akkumulator. Des Weiteren ist es denkbar, den elektrischen Energiespeicher als einen Kondensator wie beispielsweise einen Superkondensator auszuführen.

Die Vorrichtung umfasst bevorzugt mindestens ein Bedienelement, über das die Vorrichtung ein und/oder ausgeschaltet werden kann. Zusätzlich kann ein Bedienelement vorgesehen sein, über welches die Leistung der Vorrichtung gewählt werden kann. Das Bedienelement ist beispielsweise als ein Ein/AusSchalter oder als ein Taster ausgestaltet.

Das oder die Bedienelemente sind bevorzugt mit einer Steuereinheit der Vorrichtung verbunden.

Die Steuereinheit der Vorrichtung umfasst bevorzugt den Pulsgenerator zur Bereitstellung eines Ansteuersignals bzw. der elektrischen Anregungspulse, welches der Eingangsseite des Transformators zugeführt wird. Hierzu verfügt die Steuereinheit über eine Verbindung zum elektrischen Energiespeicher. Alternativ dazu können die Steuereinheit und der Pulsgenerator auch als separate Komponenten ausgestaltet sein.

Der Pulsgenerator ist beispielsweise als eine Spannungsquelle ausgestaltet, welche unter Verwendung eines Halbleiterschalters, beispielsweise ein MOSFET, kontrolliert ein- und ausgeschaltet wird. Dabei wird beispielsweise der Halbleiterschalter für die Zeitdauer des Anregungspulses leitend geschaltet, und in der folgenden Anregungspause sperrend geschaltet.

Der Pulsgenerator kann alternativ beispielsweise als ein Arbitrary-Funktionsgenerator ausgeführt sein, über den beliebige Signalformen erzeugt werden können. Des Weiteren kann die Steuereinheit einen Verstärker umfassen, um die von dem Pulsgenerator erzeugten elektrischen Anregungspulse zu verstärken.

Es kann vorgesehen sein, die Steuereinheit und die Sicherheitseinrichtung als eine gemeinsame Einheit auszugestalten. Alternativ dazu sind die Steuereinheit und die Sicherheitseinrichtung als getrennte Einheiten ausgestaltet.

Das Behandlungsinstrument der Vorrichtung dient dazu, die erzeugten hochfrequenten elektrischen Pulse bzw. deren elektrische Felder zu dem zu behandelnden Objekt oder zu dem zu behandelnden organischen bzw. biologischen Material zu leiten. Das Behandlungsinstrument umfasst einen geschlossenen Körper aus einem elektrisch isolierenden Material und eine im Inneren des Körpers angeordneten Elektrode. Im Inneren des Körpers der Elektrode ist ein Gas oder Gasgemisch aufgenommen und das Behandlungsinstrumentist für eine Gasentladung bei elektrischer Anregung durch die elektrischen Pulse eingerichtet. Im Betrieb der Vorrichtung wird das aufgenommene Gas über eine Gasentladung angeregt. Die elektrischen Felder bzw. ein resultierender dielektrischer Verschiebestrom kann somit ausgehend von der Elektrode über das angeregte Gas auf den Körper des Behandlungsinstruments geleitet werden. Der dielektrische Verschiebestrom bzw. die elektrischen Felder können dann auf ein Objekt übertragen werden, welches das Behandlungsinstrument berührt.

Das Behandlungsinstrument weist ein erstes Ende auf, welches für eine Kopplung mit der Ausgangsseite des Transformators eingerichtet ist, und weist ein zweites Ende auf, welches zum Kontaktieren von Oberflächen von Objekten und/oder von biologischem Gewebe eingerichtet ist.

Der Körper des Behandlungsinstruments ist bevorzugt im Wesentlichen länglich geformt, wobei vorgesehen ist, dass das zweite Ende größere Abmessungen aufweist als das erste Ende.

Das elektrisch isolierende Material des Körpers des Behandlungsinstruments ist bevorzugt ein Glas. Das Glas ist bevorzugt derart ausgewählt, dass es für einen Kontakt mit verletzter Haut eines Patienten geeignet ist. Das heißt, das Glas darf keine giftigen Stoffe abgeben und darf keine Irritationen oder Allergien auslösen. Geeignete Gläser sind beispielsweise Borosilikatglas, Quarzglas oder Kalk-Natron-Glas.

Das Behandlungsinstrument umfasst bevorzugt am ersten Ende, welches für die Kopplung mit der Ausgangsseite des Transformators eingerichtet ist, eine Stromdurchführung und eine Abschlusskappe. Die Abschlusskappe ist bevorzugt aus einem Kunststoff gefertigt. Der Kunststoff ist bevorzugt ausgewählt aus Polytetrafluorethylen (PTFE), Polyethylen (PE), Polystyrol (PS), Polyethylen (PE), Polypropylen (PP), Polyamid (PA) und Acrylnitril-Butadien-Styrol-Copolymer (ABS), wobei Polypropylen besonders bevorzugt ist.

Der Kunststoff der Abschlusskappe ist bevorzugt mit Fasern verstärkt, wobei die Fasern bevorzugt ausgewählt sind aus Glasfasern, Kohlenstofffasern und Aramidfasern, wobei Glasfasern besonders bevorzugt sind. Der Faservolumenanteil der Fasern liegt bevorzugt im Bereich von 20% bis 50% und besonders bevorzugt im Bereich von 30% bis 40%. Des Weiteren werden Langfasern gegenüber Kurzfasern bevorzugt. Als Langfasern werden Fasern mit einer Länge im Bereich von 1 mm bis 50 mm angesehen. Bei kürzeren Fasern handelt es sich um Kurzfasern, bei längeren Fasern um Endlosfasern.

Die am ersten Ende des Behandlungsinstruments vorgesehene Stromdurchführung weist an einer Außenseite einen elektrischen Kontakt auf und stellt eine elektrische Verbindung zur Elektrode im Inneren des Körpers des Behandlungsinstruments her.

Die Elektrode ist im Inneren des Körpers des Behandlungsinstruments bevorzugt an das erste Ende angrenzend angeordnet und ist bevorzugt stiftförmig ausgestaltet.

Die Stiftform der Elektrode weist bevorzugt eine Länge im Bereich von 10 mm bis 13 mm auf, wobei eine Länge im Bereich von 11 mm bis 12 mm besonders bevorzugt wird. Ein Durchmesser der Elektrode liegt bevorzugt im Bereich von 0,1 bis 3 mm, besonders bevorzugt im Bereich von 0,5 mm bis 2 mm. Beispielsweise beträgt der Durchmesser der Elektrode 1 mm.

Das Material der Elektrode ist bevorzugt ein Metall. Das Metall ist dabei bevorzugt ausgewählt aus Kupfer, Edelstahl, Nickel, Titan Platin und entsprechende Legierungen. Des Weiteren kann die Elektrode beschichtet sein.

Bevorzugt grenzt an das erste Ende des Behandlungsinstruments ein Stegbereich mit einem ersten Durchmesser und an das zweite Ende des Behandlungsinstruments grenzt bevorzugt ein Kolbenbereich mit einem zweiten Durchmesser an, wobei der zweite Durchmesser größer ist als der erste Durchmesser und wobei der Kolbenbereich mindestens ein Drittel der Gesamtlänge des Kolbens einnimmt.

Bevorzugt nimmt das Volumen des Kolbenbereichs mindestens zwei Drittel des Gesamtvolumens des Behandlungsinstruments ein.

Bevorzugt umfasst der Körper des Behandlungsinstruments genau zwei Bereiche, nämlich den Kolbenbereich und den Stegbereich, wobei diese nicht sprunghaft ineinander übergehen müssen sondern über einen kontinuierlichen Übergang verbunden sein können. Der Kolbenbereich weist bevorzugt eine Länge von 40 mm bis 80 mm auf, wobei eine Länge von 50 mm bis 60 mm bevorzugt wird. Beispielsweise weist der Kolbenbereich eine Länge von 58 mm auf. Der Durchmesser des Kolbenbereichs beträgt bevorzugt im Bereich von 15 mm bis 25 mm, bevorzugt von 18 mm bis 22 mm und beträgt beispielsweise 20 mm.

Das zweite Ende des Körpers ist bevorzugt im Wesentlichen plan ausgestaltet, so dass eine kreisrunde Fläche den Kolbenbereich abschließt. Diese Fläche kann über einen kontinuierlichen Übergang oder über eine scharfe Kannte in den kreiszylinderförmigen Kolbenbereich übergehen.

Für einen Kontakt mit einem zu behandelnden Objekt bzw. mit zu behandelndem organischem Material kann sowohl die im Wesentlichen plane Fläche als auch die gebogene Wandung des Kolbenbereichs verwendet werden.

Der Stegbereich des Körpers des Behandlungsinstruments weist bevorzugt eine Länge von 30 mm bis 60 mm auf, wobei eine Länge von 40 mm bis 50 mm bevorzugt wird. Beispielsweise weist der Kolbenbereich eine Länge von 45 mm auf. Der Durchmesser des Stegbereichs beträgt bevorzugt im Bereich von 5 mm bis 15 mm, bevorzugt von 8 mm bis 12 mm und beträgt beispielsweise 10 mm.

Die Abschlusskappe umschließt den Körper des Behandlungsinstruments am ersten Ende, wobei eine elektrische Kontaktfläche am ersten Ende frei bleibt. Die Abschlusskappe umfasst bevorzugt einen Hülsenbereich, welcher sich über zumindest einen Teil des Stegbereichs ausgehend vom ersten Ende erstreckt. Die Länge des Hülsenbereichs beträgt bevorzugt im Bereich von 10 mm bis 50 mm, besonders bevorzugt im Bereich von 15 bis 30 mm. Beispielsweise ist der Hülsenbereich 20 mm lang. Der Durchmesser der Abschlusskappe beträgt bevorzugt im Bereich von 7 mm bis 18 mm, bevorzugt von 10 mm bis 15 mm. Beispielsweise ist der Durchmesser der Abschlusskappe 12 mm.

Die Abschlusskappe ist bevorzugt über einen Klebstoff, welcher im Hülsenbereich angeordnet ist, mit dem Körper des Behandlungsinstruments verbunden. Der Klebstoff ist bevorzugt ein temperaturbeständiger Epoxidharzkleber.

Der Körper des Behandlungsinstruments ist geschlossen, so dass dieser gasdicht ist. Der Körper des Behandlungsinstruments ist mit einem Edelgas oder einem Edelgasgemisch gefüllt, welches mit Bezug zum Umgebungsdruck von normalerweise etwa 1 bar einen reduzierten Druck aufweist. Bevorzugt liegt der Druck der Gasfüllung des Behandlungsinstruments in einem Bereich von 0,001 mbar bis 1 mbar, besonders bevorzugt im Bereich von 0,01 bis 0,7 mbar.

Das Behandlungsinstrument kann beispielsweise mit einem Edelgas wie Neon oder Argon oder einer Mischung aus mehreren Edelgasen gefüllt sein. Die Gasmischung kann auch weitere Gase wie beispielsweise Stickstoff enthalten, beispielsweise eine Mischung aus Neon, Argon und Stickstoff. Bevorzugt wird Neon für die Gasfüllung verwendet. Mit Neon werden beispielsweise im Vergleich zu einer Füllung mit Argon höhere Feldstärken der elektrischen und magnetischen Felder erreicht.

Der Körper des Behandlungsinstruments ist bevorzugt einwandig ausgestaltet, so dass nur eine einzige Glasschicht das Innere des Behandlungsinstruments, welches mit dem Edelgas oder dem Edelgasgemisch gefüllt ist, von der Umgebung trennt.

Das Behandlungsinstrument der Vorrichtung ist bevorzugt auswechselbar ausgestaltet, um diese bei Bedarf ersetzen oder reinigen zu können. Durch Trennen des Behandlungsinstruments kann dieses insbesondere auch einfach sterilisiert werden.

Bevorzugt umfasst die Vorrichtung eine Sicherheitseinrichtung, welche die Anwesenheit des Behandlungsinstruments erkennt und erkennen und bei Abwesenheit des Behandlungsinstruments eine Energiezufuhr zum Transformator zu unterbricht. Hierzu kann die Sicherheitseinrichtung einen Detektor umfassen, welcher beispielsweise als Mikroschalter oder als Lichtschranke eingerichtet ist. Ein als Mikroschalter ausgebildeter Detektor ist beispielsweise derart angeordnet, dass dieser bei vorhandenem Behandlungsinstrument gedrückt wird. Ein als Lichtschranke ausgebildeter Detektor ist beispielsweise derart angeordnet, dass ein Lichtstrahl durch das anwesende Behandlungsinstrument unterbrochen wird. Eine Abschlusskappe des Behandlungsinstruments kann in diesem Zusammenhang insbesondere Lichtundurchlässig ausgestaltet sein, um den Lichtstrahl der Lichtschranke zu unterbrechen.

Bevorzugt weist das Behandlungsinstrument einen Datenspeicher auf, der bei in die Vorrichtung eingesetztem Behandlungsinstrument von einem Lesegerät der Vorrichtung auslesbar ist. Der Datenspeicher kann beispielsweise optisch in Form eines eindimensionalen Barcodes oder in Form eines Matrixcodes wie eines QR Codes ausgebildet sein. Alternativ dazu kann der Datenspeicher beispielsweise als ein über ein drahtloses Kommunikationsverfahren wie RFID auslesbarer Speicherchip ausgebildet sein.

Der Datenspeicher des Behandlungsinstruments kann beispielsweise Angaben zum Typ und/oder den erforderlichen Betriebsparametern sowie Angaben für einzuhaltende Grenzwerte enthalten. Die Einhaltung der Grenzwerte kann dann insbesondere durch eine entsprechend eingerichtete Sicherheitseinrichtung kontrolliert werden. Die einzuhaltenden Grenzwerte können insbesondere Obergrenzen und/oder Untergrenzen für Spannung, Frequenz und/oder Stromstärke der hochfrequenten elektrischen Pulse enthalten.

Um eine einfache und sichere Reinigung des Behandlungsinstruments durch eine Autoklavierung mit Wasserdampf zu ermöglichen, ist das Behandlungsinstrument bevorzugt eingerichtet, für Wasserdampf bei einer Temperatur im Bereich von 110°C bis 140°C beständig zu sein. Die verwendeten Materialien, also insbesondere das Glas des Körpers sowie der Kunststoff der Abschlusskappe sowie gegebenenfalls eingesetzte Verbindungsmittel wie beispielsweise ein Klebstoff, sind entsprechend ausgewählt. Gegenüber üblichen Abschlusskappen aus einem Metall weist die vorgeschlagene Abschlusskappe aus einem Kunststoffmaterial günstige thermische Eigenschaften und eine gute Hydrolysebeständigkeit auf.

Um eine einfache elektrische Kopplung des Behandlungsinstruments zu gewährleisten, ist das Behandlungsinstrument bevorzugt über eine Steckverbindung aufgenommen, wobei das Behandlungsinstrument über einen Kontakt der Steckverbindung elektrisch mit der Ausgangsseite des Transformators verbunden ist. Der Kontakt der Steckverbindung ist beispielsweise als ein Federkontakt ausgestaltet, welcher bei eingesetztem Behandlungsinstrument gegen die elektrische Kontaktfläche des Behandlungsinstruments vorgespannt ist.

Bevorzugt weist das Behandlungsinstrument einen Hülsenbereich auf, welcher den Stegbereich des Behandlungsinstruments zumindest teilweise umschließt, und die Steckverbindung weist eine Klemmeinrichtung auf, welche eingerichtet ist, das Behandlungsinstrument an dem Hülsenbereich zu halten.

Ein weiterer Aspekt der Erfindung betrifft ein Verfahren zur Inaktivierung von Mikroorganismen, wobei eine der beschriebenen Vorrichtungen bereitgestellt wird und das Behandlungsinstrument der Vorrichtung mit hochfrequenten elektrischen Pulsen angeregt wird. Ferner ist vorgesehen, dass das Behandlungsinstrument auf eine Oberfläche eines Objekts, bei dem Mikroorganismen inaktiviert werden sollen, aufgesetzt wird, so dass der Körper des Behandlungsinstrument die Oberfläche berührt, und das Behandlungsinstrument über die Oberfläche des Objekts geführt wird. Dabei ist die Gehäuseabschirmung der Vorrichtung elektrisch von Objekt getrennt, so dass keine elektrisch leitende Verbindung zwischen dem Objekt und den zweiten Anschlüssen des Transformators hergestellt wird.

Insbesondere sind die Vorrichtung und das Objekt, bei dem Mikroorganismen inaktiviert werden sollen, nicht mit einer gemeinsamen Erde verbunden, das Objekt wird bei dem Verfahren ausschließlich mit einer einzigen Elektrode kontaktiert, welche hier der Körper des Behandlungsinstruments der Vorrichtung ist.

Die beschriebenen Vorrichtungen sind bevorzugt zur Verwendung mit den hier beschriebenen Verfahren eingerichtet, so dass im Zusammenhang mit den Verfahren beschriebene Merkmale auch entsprechend für die Vorrichtungen und umgekehrt im Rahmen der Vorrichtungen beschriebene Merkmale für die Verfahren gelten.

Bevorzugt ist das Objekt ausgewählt aus medizinischen Instrumenten und organischem bzw. biologischem Material. Bei dem organischen Material handelt es sich beispielsweise um Haut oder um eine Gewebeprobe. Entsprechend kann das Verfahren insbesondere für nicht-therapeutische Behandlungen angewendet werden.

Das Verfahren eignet sich auch für die Anwendung an einem Patienten, beispielsweise zur Inaktivierung von Mikroorganismen im Bereich von Wunden und/oder zur Unterstützung der Wundheilung. Durch Besiedelung mit Keimen kommt es oft zu dem Problem, dass Wunden schlecht heilen. Handelt es sich dabei um einen gegen ein oder mehrere Antibiotika resistenten Keim, ist die Versorgung der Wunde häufig besonders aufwendig und langwierig. Bei Anwendung des vorgeschlagenen Verfahrens werden auch resistente Keime abgetötet und eine Wundheilung gefördert. Zur Anwendung bei der Behandlung einer Wunde wird die vorgeschlagene Vorrichtung bereitgestellt und das Behandlungsinstrument der Vorrichtung wird mit hochfrequenten elektrischen Pulsen angeregt. Das Behandlungsinstrument wird dann bevorzugt mit der Haut bzw. der Wunde in direkte Verbindung gebracht, so dass das Behandlungsinstrument den Patienten berührt und einmal oder auch mehrfach über die gesamte Oberfläche der Wunde geführt, wobei jeder Bereich der Oberfläche der Wunde bevorzugt im Bereich von einer Minute bis 10 Minuten, besonders bevorzugt im Bereich von 2 bis 6 Minuten in direktem Kontakt mit dem Behandlungsinstrument ist. Die elektrischen Parameter der hochfrequenten elektrischen Pulse sind hierzu beispielsweise wie folgt gewählt: 20 kV Spannung (Spitze-Spitze), 55 kHz Frequenz, 280 Hz Wiederholrate und 128 µs Pulsbreite.

Alternativ hierzu ist es möglich, die zu behandelnde Wunde abzudecken bzw. einen bereits vorhandenen Verband bei der Behandlung bestehen zu lassen und somit nicht zu entfernen. In diesem Fall wird das Behandlungsinstrument der Vorrichtung für eine Behandlung der Wunde direkt auf eine Wundabdeckung bzw. den Verband aufgesetzt, so dass diese die Wundabdeckung bzw. den Verband berührt, und einmal oder mehrfach über die gesamte Oberfläche der Wunde geführt. Bei der Wundabdeckung kann es sich beispielsweise um eine übliche Wundkompresse handeln. Die elektrischen Parameter der hochfrequenten elektrischen Pulse sind hierzu beispielsweise wie folgt gewählt: 29 kV Spannung (Spitze-Spitze), 55 kHz Frequenz, 280 Hz Wiederholrate und 156 µs Pulsbreite.

Bei der Wundbehandlung kann die Vorrichtung entweder von einem zu behandelnden Patienten selbst oder von einer weiteren Person geführt werden. Bei der Behandlung von entnommenen Gewebeproben oder anderem organischem bzw. biologischem Material wird entsprechend verfahren.

Das Verfahren eignet sich des Weiteren insbesondere zur Sterilisierung bzw. Desinfizierung von medizinischen Instrumenten. Dazu wird die vorgeschlagene Vorrichtung bereitgestellt und das Behandlungsinstrument der Vorrichtung wird mit hochfrequenten elektrischen Pulsen angeregt. Das Behandlungsinstrument wird dann mit der Oberfläche des zu desinfizierenden medizinischen Instruments in direkte Verbindung gebracht, so dass das Behandlungsinstrument das medizinische Instrument direkt berührt und einmal oder auch mehrfach über die gesamte Oberfläche des Instruments geführt, wobei jeder Bereich der Oberfläche bevorzugt im Bereich von einer Minute bis 10 Minuten, besonders bevorzugt im Bereich von 2 bis 6 Minuten in direktem Kontakt mit dem Behandlungsinstrument ist. Bei der Desinfizierung von beliebigen anderen Objekten bzw. Gegenständen wird entsprechend verfahren. Die elektrischen Parameter der hochfrequenten elektrischen Pulse sind hierzu beispielsweise wie folgt gewählt: 34 kV Spannung (Spitze-Spitze), 55 kHz Frequenz, 280 Hz Wiederholrate und 170 µs Pulsbreite.

Es wird davon ausgegangen, dass die von dem Behandlungsinstrument ausgehende Wirkung auf der Einwirkung der elektrischen Felder beruht. Die elektrischen Felder und die von diesen bewirkten dielektrischen Verschiebeströme wirken nicht nur auf die Oberfläche ein, die mit dem Behandlungsinstrument in Kontakt steht, sondern wirken auch in dem darunter liegenden Material, so dass das Objekt auch im Inneren behandelt wird und Mikroorganismen inaktiviert werden.

Es wird davon ausgegangen, dass die Wirkung des Behandlungsinstruments auf Keime, wie beispielsweise Bakterien, darauf beruht, dass durch die gepulsten elektrischen Felder eine Zellmembran der Bakterien dauerhaft geschädigt wird. Bakterien, welche sich auf der Oberfläche oder innerhalb eines Objekts befinden, werden bei der Behandlung mit der Vorrichtung geschädigt und in der Folge abgetötet. Dabei wird das Objekt nicht wesentlich erwärmt und auch keiner nennenswerten Dosis UV-Strahlung ausgesetzt, so dass das Verfahren sehr schonend arbeitet.

Ferner wurde beobachtet, dass bei Behandlung einer Wunde mit der beschriebenen Vorrichtung die Wundheilung in einem Maße gefördert wird, welches über die rein desinfizierende Wirkung hinausgeht. Es wird davon ausgegangen, dass die von dem Behandlungsinstrument ausgehenden dielektrischen Verschiebungsströme die Wundheilungsprozesse stimulieren.

Die von dem Behandlungsinstrument ausgehenden elektrischen Felder werden durch Materie, welche sich in der Nähe befindet, beeinflusst. Dabei werden die elektrischen Felder insbesondere durch die Anwesenheit von elektrisch leitfähigen Materialien konzentriert. Für eine besonders gute Einwirkung auf kleine, tragbare Objekte ist es daher bevorzugt, wenn diese von einer Person, welche die Behandlung durchführt, in der Hand gehalten werden, während das Behandlungsinstrument über die Oberfläche des Objekts geführt wird. Der Körper der Bedienperson weist eine gute elektrische Leitfähigkeit auf und sorgt somit dafür, dass die elektrischen Felder vorteilhaft am in der Hand gehalten Objekt konzentriert werden.

Die an der Oberfläche des Behandlungsinstruments bei elektrischer Anregung des Behandlungsinstrument auftretenden elektrischen Felder weisen bevorzugt eine Feldstärke im Bereich von 500 V/m bis 6000 V/m, besonders bevorzugt von 500 V/m bis 1800 V/m auf.

Eine magnetische Flussdichte an der Oberfläche des Behandlungsinstruments liegt beispielsweise im Bereich von 10µT bis 73 µT, bevorzugt im Bereich von 10 µT bis 43µt.

### In vitro Versuche

Es wurde die Wirkung der Vorrichtung bzw. des Verfahrens auf Testkeime untersucht. Als Testkeime wurden a) NCTC 12493 Staphylococcus aureus und b) ATCC 27853 Pseudomonas aeruginosa untersucht. Hierzu wurde analog zu dem Hemmhoftest zur Prüfung der Empfindlichkeit eines Bakteriums gegenüber einem Antibiotikum vorgegangen. Der jeweilige Testkeim wurde dazu auf einen Agarmedium aufgetragen und anschließend wurde das Agarmedium entweder mit dem erfindungsgemäßen Verfahren behandelt oder nicht. Der Erfolg der Behandlung wird im Vergleich zu dem unbehandelten Agarmedium durch eine Hemmung des Wachstums der Keime sichtbar.

Der Testkeim a) NCTC 12493, Staphylococcus aureus, ist ein kugelförmiges, grampositives Bakterium. Staphylokokken bewegen sich nicht aktiv und bilden keine Sporen. Staphylococcus aureus ist weit verbreitet, kommt in vielen Habitaten vor, lebt meistens als harmloser, beim Menschen zur normalen Besiedlungsflora der Haut und Schleimhaut gehörender Saprobiont und Kommensale, kann aber auch pathogen sein und neben Haut- und Weichgewebsinfektionen auch Lungenentzündung, Hirnhautentzündung, Endokarditis und sogar ein toxisches Schocksyndrom und Sepsis verursachen.

Der Testkeim b) ATCC 27853 Pseudomonas aeruginosa (von lat. aerugo Grünspan)
ist ein gramnegatives, oxidasepositives Stäbchenbakterium der Gattung Pseudomonas. Es wurde im Jahr 1900 von Walter Migula entdeckt. Die Namensgebung bezieht sich dabei auf die blau-grüne Färbung des Eiters bei eitrigen Infektionskrankheiten.

Bei den Testkeimen handelt es sich um wichtige Krankenhauskeime, die auch gegen mehrere Antibiotika Resistenzen entwickelt haben.

Die Testkeime wurden jeweils auf vorbereitete Agarplatten mit einem Durchmesser von 70 mm aufgebracht, um eine Wunde mit Wundexsudat zu simulieren. Anschließend wurden die Agarplatten unter Verwendung der beschriebenen Vorrichtung mit den in Tabelle 1 angegebenen Parametern behandelt. Die Behandlungsdauer wurde dabei wie in Tabelle 2 angegeben variiert, wobei bei den Vergleichsversuchen 5 und 6 keine Behandlung durchgeführt wurde. Während der angegebenen Behandlungsdauer wurde das Behandlungsinstrument der Vorrichtung auf die Agarplatte aufgesetzt und über die Oberfläche der Agarplatte bewegt. Während der Behandlungszeit war das Behandlungsinstrument die gesamte Zeit in Kontakt mit der Oberfläche der Agarplatte. Danach wurden die Agarplatten mindestens 48 Stunden in einem Inkubator behandelt. Nach 48 Stunden erfolgte eine erste Bewertung. Je nach Ergebnis erfolgte eine weitere Behandlung im Inkubator für weitere 48 Stunden.

Zum Nachweis einer Besiedlung wird die Oberfläche der Agarplatten mit einer sterilen Ringschlinge abgestrichen und anschließend die Ringschleife begutachtet.

Bei den Versuchen wurde die Vorrichtung mit folgenden Parametern betrieben:

**(Tabelle 1)**

| Parameter | Wert |
|---|---|
| | |
| Spannung (Spitze-Spitze an Ausgangsseite): | 23 kV |
| Frequenz an Ausgangsseite: | 25,9 kHz (bei Kontakt des Behandlungsinstruments mit Objekt) |
| | 60 kHz (kein Kontakt) |
| Pulswiederholrate: | 300 Hz |
| Maximaler Strom an Ausgangsseite | 80 µA |

Die Versuchsergebnisse sind in Tabelle 2 aufgetragen.

**(Tabelle 2)**

| Nr. | Keim | Behandlungsdauer | Ergebnis nach 48h | Ergebnis nach 96 h |
|---|---|---|---|---|
| 1 | a) | 2 min | Hemmhof ca. 30% | ca. 30% |
| 2 | b) | 2 min | Hemmhof ca. 40% | ca. 40% |
| 3 | a) | 6 min | Hemmhof ca. 90% | ca. 90% |
| 4 | b) | 6 min | Hemmhof ca. 90% | ca. 90%* |
| 5 | a) | (unbehandelt) | Kein Hemmhof | Kein Hemmhof, besiedelte Fläche noch größer |
| 6 | b) | (unbehandelt) | Kein Hemmhof | Kein Hemmhof, besiedelte Fläche noch größer |

Die in der Spalte "Ergebnis" angegebenen %-Angaben beziehen sich auf den Prozentsatz der Fläche der Agarplatte, welche keine Bakterienbesiedlung und damit einen Hemmhof aufwies. Bei den Versuchen 3 und 4 wurde eine praktisch vollständige Hemmung des Bakterienwachstums erzielt. Die verbleibende besiedelte Fläche von 10% und weniger der Gesamtfläche der Agarplatte wird darauf zurückgeführt, dass an den Rändern der Plätte aufgrund der Wände der verwendeten Petrischale das Behandlungsinstrument die Ränder nicht vollständig überstrichen hat. Bei den Versuchen 3 und 4 wird somit von einer vollständigen Hemmung des Bakterienwachstums für die mit dem Behandlungsinstrument behandelte Oberfläche der Agarplatte ausgegangen.

Bei den Versuchen 1 und 2 wurde keine vollständige Hemmung des Bakterienwachstums erzielt. Dies wird darauf zurückgeführt, dass die kurze Behandlungsdauer von nur 2 Minuten nicht für ein vollständiges Überstreichen der gesamten Oberfläche der Agarplatte ausreichend war und somit große Bereiche der Agarplatte nicht durch Überstreichen mit dem Behandlungsinstrument behandelt wurden.

Um die Verträglichkeit auch bei Anwendung am menschlichen Körper zu testen, wurde eine Erwärmung der Haut untersucht. Hierzu wurde das Behandlungsinstrument im Bereich der Hand auf die Haut einer Versuchsperson aufgesetzt und mit elektrischen Pulsen gemäß den Parametern in Tabelle 1 angeregt. Die Erwärmung der Haut wurde über ein Infrarotthermometer überwacht. Die Ausgangstemperatur vor Beginn der Behandlung betrug 32°C. Nach 6 Minuten konstantem Hautkontakt stieg die Temperatur um 1° auf 33°C. Die für Medizinprodukte geforderten Richtwerte, nämlich eine Erhöhung von nicht mehr als 5° und nicht über 41°C werden eingehalten und sogar deutlich unterschritten.

Zur Verträglichkeit wurde zudem untersucht, ob bei der Gasentladung in dem Behandlungsinstrument UV-Strahlung in einem unzulässigen Maß entsteht. Bei einem Versuch mit den in Tabelle 1 angegebenen Parametern wurde direkt angrenzend an den Kolbenbereich des Behandlungsinstruments eine UV-Leistung von 0,035 mW/cm² gemessen. Der Richtwert von 0,1 mW/cm² wurde eingehalten.

Bei den Versuchen an der Versuchsperson konnte keine schädliche Einwirkung des Behandlungsinstruments auf menschliche Zellen nachgewiesen werden.

### Anwendung der Vorrichtung an Brandwunden

Bei einem Patienten mit Brandwunden im Bereich der Kopfhaut, des Kinns (Hals-Thorax), der Brüste und der Hände wurden die Brandwunden zunächst konventionell gepflegt. Die betroffenen Hautareale, Kopf-Hals-Thorax und Brüste sowie beide Hände hatten einen Verbrennungsgrad IIb bis III.

Es lag eine tangentiale Nekrektomie an allen betroffenen Hautrealen vor. In der Folge wurden die Wunden an Händen und Unterarmen über Débridement und Spalthauttransplantation von den Oberschenkeln versorgt. Bei den Brüsten wurde beidseitig, Débridement und Integra - Transplantation durchgeführt. Gesicht und Kopf wurden mit einem Feuchtverband versorgt.

Außer den Brandwunden auf dem Kopf und am Hals waren die Heilungstendenzen gut bzw. im normalen Verlauf. In einer direkt anschließenden, stationären Reha wurde mit konservativer Behandlung fortgefahren, mittels dexpanthenolhaltiger Salbenverbände.

Der Zustand des Patienten nach drei Monaten konventioneller Behandlung stellte sich wie folgt dar:
Nach erfolglosem Wundverschluss auf dem Kopf und einer sich entzündeten Halswunde wurde begonnen, die Wundbehandlung durch Anwenden der beschriebenen Vorrichtung zu unterstützen. Zu diesem Zeitpunkt war die Wunde im Bereich der Kopfhaut nicht geschlossen. Die Wunde im Bereich des Kinns wies eine Entzündung mit Eiterbildung auf. Die Wunden an den Händen waren geschlossen, jedoch waren die Wunden rissig und das Narbengewebe nicht geschmeidig.

Die Wunden im Bereich der Kopfhaut und des Kinnbereichs wurden jeweils am Vormittag und am Nachmittag 3 mal für 2 Minuten mit der erfindungsgemäßen Vorrichtung behandelt. Die Wunden wurden vor der Behandlung mit einer Mullkompresse abgedeckt. Das Behandlungsinstrument der Vorrichtung wurde anschließend in direkten Kontakt mit der Mullkompresse gebracht und über den Bereich der Wunde geführt, so dass die gesamte Fläche der Wunde behandelt wurde. Dabei wurde das Behandlungsinstrument nach dem Auflegen auf die Wunde bzw. die Kompresse ca. 30 Sekunden nicht bewegt.

Nach ca. 30 Sekunden wurde das Behandlungsinstrument ca. 15 Sekunden über die Wunde geführt. In der Folge wurde wieder das Behandlungsinstrument mit einem neuen Schwerpunkt auf die Wunde gelegt und dann wieder ca. 15 Sekunden über die Wunde geführt. Die Behandlung von 2 Minuten wurde 3 mal wiederholt.

Bei der ersten und zweiten Behandlung erfolgte die Behandlung mit der Stufe 4 der Vorrichtung, wobei die verwendeten elektrischen Parameter für die hochfrequenten elektrischen Pulse wie folgt waren: 20 kV Spannung (Spitze-Spitze), 45 kHz Frequenz, 280 Hz Wiederholrate.

Bei der dritten Behandlung erfolgte die Behandlung mit der Stufe 2 der Vorrichtung, wobei die verwendeten elektrischen Parameter für die hochfrequenten elektrischen Pulse wie folgt waren: 9,5 kV Spannung (Spitze-Spitze), 45 kHz Frequenz, 280 Hz Wiederholrate.

Bereits nach 8 Behandlungstagen hatte sich die Brandwunde im Bereich der Kopfhaut nahezu vollständig geschlossen.

Die Behandlung wurde fortgesetzt, wobei die nächsten 14 Tage jeweils eine Behandlung vormittags und eine am Abend ohne eine Kompresse mit der Stufe 2.

Die Wunde im Halsbereich war nach 21 Tagen vollständig geheilt.

Die Wunde an der Kopfhaut wurde für weitere 30 Tage behandelt mit der Stufe 2 behandelt, wobei eine Behandlung täglich erfolgte. In besonderen Fällen, beispielsweise um die Wunde nach einer Dusche zu reinigen, wurde die Stufe 4 angewendet. Nach den weiteren 30 Tagen (ca. drei Monaten) der Behandlung war die Wunde im Bereich der Kopfhaut nicht nur vollständig geschlossen, sondern es trat überraschenderweise Haarwuchs auf, so dass die Funktion der Kopfhaut vollständig wiederhergestellt wurde. Es wird davon ausgegangen, dass die von dem Behandlungsinstrument ausgehenden elektrischen und/oder magnetischen Wechselfelder in der Dermis die biologische Wirkung ausgelöst hat, unter anderem den Haarwuchs wieder anzuregen und die darüber liegende Epidermis so hergerichtet hat, dass Haar aus der Brandwunde wachsen konnte.

Die Wunde im Kinnbereich war nach 8 Behandlungstagen frei von Entzündungen und nach 23 Behandlungstagen zwar noch schwach sichtbar, aber ansonsten vollständig verheilt.

Die Wunden im Bereich der Hände waren zu Behandlungsbeginn bereits geschlossen, so dass für die Behandlung mit der erfindungsgemäßen Vorrichtung keine Wundabdeckung erforderlich war. Das Behandlungsinstrument der Vorrichtung wurde für die Behandlung in direkten Kontakt mit den Wunden gebracht, wobei jeder Finger täglich einmal für 2 Minuten behandelt wurde. Die Behandlung wurde durch Anwenden einer medizinischen Hautcreme unterstützt.

Nach drei Monaten Behandlung sind die Transplantate voll funktionsfähig und die Narben sind geschmeidig. Eine innere Vernarbung trat nicht auf.

Die jeweiligen Schritte wurden immer mit einem sterilen Behandlungsinstrument aus einer sterilen Verpackung vorgenommen. Nach der Behandlung wurden die Behandlungsinstrumente gereinigt und gereinigt in einer Verpackung vakuumiert, in den Autoklaven bei 141°C sterilisiert. So waren die Behandlungsinstrumente immer steril gelagert und in der Folge steril verwendbar.

### Kurze Beschreibung der Zeichnungen

Ausführungsformen der Erfindung werden anhand der Zeichnungen und der nachfolgenden Beschreibung näher erläutert.

### Es zeigen:

Figur 1a eine schematische Darstellung der Vorrichtung zur Unterstützung der Wundheilung und/oder zur Inaktivierung von Mikroorganismen als Schnittansicht von oben,
- Figur 1b: eine schematische Schnittansicht eines Transformators,
- Figur 2: ein vereinfachtes schematisches Schaltbild der Vorrichtung,
- Figur 3: eine schematische Darstellung eines Behandlungsinstruments der Vorrichtung,
- Figur 4: eine Detailansicht des Behandlungsinstruments,
- Figur 5: eine qualitative Darstellung eines hochfrequenten elektrischen Pulses und
- Figur 6: eine qualitative Darstellung eines elektrischen Anregungspulses zur Anregung des Transformators.

### Ausführungsformen der Erfindung

In der nachfolgenden Beschreibung der Ausführungsformen der Erfindung werden gleiche oder ähnliche Elemente mit gleichen Bezugszeichen bezeichnet, wobei auf eine wiederholte Beschreibung dieser Elemente in Einzelfällen verzichtet wird. Die Figuren stellen den Gegenstand der Erfindung nur schematisch dar.

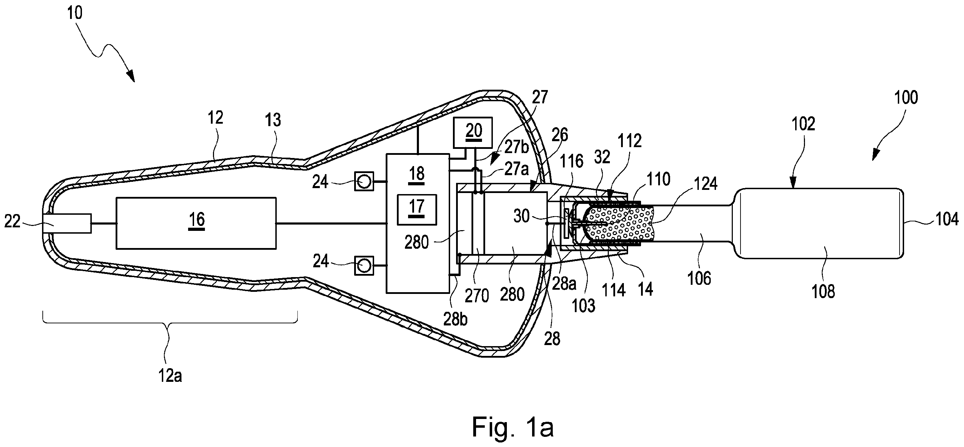

Figur 1a zeigt eine Vorrichtung 10 zur Unterstützung der Wundheilung und/oder zur Inaktivierung von Mikroorganismen. Die Vorrichtung 10 umfasst ein Gehäuse 12, welches bis auf einen Teil eines Behandlungsinstruments 100 sämtliche Komponenten der Vorrichtung 10 aufnimmt. An der Stelle, an der das Behandlungsinstrument 100 aus dem Gehäuse 12 herausragt, ist eine Instrumentenführung 14 vorgesehen. Das Gehäuse 12 mit der Instrumentenführung 14 ist aus einem elektrisch isolierenden Material wie beispielsweise einem Kunststoff gefertigt. Das Gehäuse 12 der Vorrichtung 10 ist in dem in Figur 1a gezeigten Beispiel mit einem Griffabschnitt 12a versehen, an dem die Vorrichtung 10 während einer Behandlung ergonomisch in der Hand gehalten werden kann.

Die Vorrichtung 10 umfasst einen elektrischen Energiespeicher 16, welcher eine Steuerung 18 mit elektrischer Energie versorgt. Der elektrische Energiespeicher 16 ist bevorzugt als ein Lithium-Ionen Akkumulator ausgestaltet. Zum Aufladen des elektrischen Energiespeichers 16 ist ein Ladeanschluss 22 vorgesehen, welcher über eine Öffnung im Gehäuse 12 zugänglich ist.

Die Steuerung 18 ist mit der Eingangsseite 27 eines Transformators 26 verbunden. Im Betrieb der Vorrichtung 10 erzeugt die Steuerung 18 unter Verwendung eines Pulsgenerators 17 elektrische Anregungspulse, welche der Eingangsseite 27 des Transformators 26 zugeführt werden. Der Transformator 26 erzeugt dann eine Hochspannung in Form von hochfrequenten elektrischen Pulsen, welche an einer Ausgangsseite 28 des Transformators 26 ausgegeben werden. Die Hochspannung wird verwendet, um das Behandlungsinstrument 100, welches elektrisch mit dem Transformator 26 gekoppelt ist, anzuregen. Für eine elektrische Abschirmung der im Gehäuse 12 aufgenommenen Komponenten ist eine Gehäuseabschirmung 13 angeordnet, welche in dem dargestellten Beispiel in Form einer elektrisch leitenden Folie an der Innenseite des Gehäuses 12 ausgeführt ist. Alternativ kann die Gehäuseabschirmung 13 beispielsweise auch als eine elektrisch leitfähige Beschichtung der Innenseite des Gehäuses 12 ausgeführt sein.

Die schematische Darstellung des Transformators 26 zeigt, dass eine Primärspule 270 zwischen zwei Teilen einer Sekundärspule 280 eingesetzt ist. Ein erster Anschluss 27a der Eingangsseite des Transformators 26, also der Primärspule 270, ist dabei direkt mit der Steuerung 18 und damit mit dem Pulsgenerator 17 verbunden. Ein zweiter Anschluss 27b der Primärspule 270 ist über eine Sicherheitseinrichtung 20 mit der Steuerung 18 verbunden und über die Steuerung 18 mit der Gehäuseabschirmung 13 verbunden. Ein erster Anschluss 28a der Ausgangsseite des Transformators 26, also der Sekundärspule 280, ist mit dem Behandlungsinstrument 100 verbunden. Ein zweiter Anschluss 28b der Sekundärspule 280 ist mit der Steuerung 18 und über diese mit der Gehäuseabschirmung 13 verbunden. Die zweiten Anschlüsse 27b und 28b sind dabei jeweils indirekt über die Steuerung 18 mit der Gehäuseabschirmung 13 verbunden und weisen damit das gleiche elektrische Potential auf. Die von der Gehäuseabschirmung 13 gebildete elektrische Masse ist nicht geerdet, sondern ist durch das Gehäuse 12 elektrisch isoliert.

Das Behandlungsinstrument 100 umfasst einen Körper 102 aus einem elektrisch isolierenden Material. Der Körper 102 ist hier länglich ausgestaltet und weist einen Stegbereich 106 und einen Kolbenbereich 108 auf. Das Material des Körpers 102 ist beispielsweise ein Glas. Ein erstes Ende 103 des Behandlungsinstruments 100 grenzt an den Stegbereich 106 und ein zweites Ende 104 des Behandlungsinstrument 100 grenzt an den Kolbenbereich 108 an. Der Kolbenbereich 108 ragt vollständig aus dem Gehäuse 12 der Vorrichtung 10 heraus, während der Stegbereich 106 zumindest teilweise von dem Gehäuse 12 umschlossen ist. Das Innere des Körpers 102 ist mit einem Edelgas 124 gefüllt, welches im Vergleich zum Umgebungsdruck einen reduzierten Druck aufweist.

Das Behandlungsinstrument 100 weist angrenzend an das erste Ende 103 eine Abschlusskappe 112 auf, welche aus einem Kunststoff gefertigt ist. Die Abschlusskappe 112 weist einen Hülsenbereich 114 auf, welcher einen an das erste Ende 103 angrenzenden Teil des Stegbereichs 106 umschließt. Des Weiteren ist am ersten Ende 103 eine elektrische Kontaktfläche 116 angeordnet, welche durch die Abschlusskappe 112 und den Körper 102 hindurch mit einer Elektrode 110 elektrisch verbunden ist.

Das Behandlungsinstrument 100 ist in der Vorrichtung 10 auswechselbar aufgenommen. Hierzu ist eine Klemmvorrichtung 32 vorgesehen, welche das Behandlungsinstrument 100 am Hülsenbereich 114 der Abschlusskappe 112 aufnimmt. In dieser Position wird die elektrische Kontaktfläche 116 das Behandlungsinstrument 100 mit einem elektrischen Federkontakt 30 kontaktiert, der mit der Ausgangsseite 28 des Transformators 26 verbunden ist. Das Behandlungsinstrument 100 ist somit elektrisch mit dem Transformator 26 gekoppelt, wenn das Behandlungsinstrument 100 in der Klemmvorrichtung 32 der Vorrichtung 10 aufgenommen ist.

Um die Vorrichtung 10 ein- und/oder auszuschalten und um ein Ändern von Betriebsparametern zu ermöglichen, sind Bedienelemente 24 vorgesehen, welche mit der Steuerung 18 verbunden sind. Die Bedienelemente 24 sind beispielsweise als Taster ausgestaltet. Nach Einschalten der Vorrichtung 10 beginnt die Steuerung 18 damit, elektrische Anregungspulse niedriger Spannung zu erzeugen, welche dem Transformator 26 an dessen Eingangsseite 27 als Ansteuersignal zugeführt wird. Die elektrischen Anregungspulse werden durch den Transformator in hochfrequente elektrische Pulse mit hoher Spannung transformiert und als Anregungssignal zur Anregung des Behandlungsinstruments 100 verwendet. Hierbei wird das im Inneren des Körpers 102 des Behandlungsinstruments 100 aufgenommene Edelgas 124 zu einer Gasentladung angeregt. Des Weiteren entstehen starke elektrische Wechselfelder.

Die Funktion der Vorrichtung 10 wird bevorzugt überwacht. Dazu umfasst die Vorrichtung 10 in der in Figur 1a dargestellten Ausführungsform eine Sicherheitseinrichtung 20, welche in dieser Ausführungsform als eine zusätzliche Komponente ausgestaltet ist. Alternativ dazu könnte die Sicherheitseinrichtung 20 auch als Teil der Steuerung 18 ausgestaltet werden. Die Sicherheitseinrichtung 20 ist dazu ausgestaltet und eingerichtet, die Hochspannung an der Ausgangsseite 28 des Transformators 26, also die hochfrequenten elektrischen Pulse, darauf zu überwachen, dass vorgegebene Grenzwerte für elektrische Parameter nicht überschritten bzw. unterschritten werden. Die elektrischen Parameter umfassen beispielsweise den elektrischen Strom, die Spannung, eine Pulswiederholrate, eine Pulsbreite und eine Pulsfrequenz. In der in Figur 1a dargestellten Ausführungsform wird die Messung an der Eingangsseite 27 des Transformators 26 durchgeführt, wobei ausgenutzt wird, dass die hochfrequenten elektrischen Pulse nach Abklingen des elektrischen Anregungspulses ein Signal an der Eingangsseite 27 des Transformators 26 erzeugen, welches gemessen werden kann. Für eine Messung dieser elektrischen Parameter ist in dem dargestellten Beispiel der zweite Anschluss 27b der Eingangsseite 27 des Transformators 26 über die Sicherheitseinrichtung 20 mit der Steuerung 18 verbunden. Werden die Grenzwerte über- bzw. unterschritten, wird die Energiezufuhr zum Transformator 26 durch die Steuerung 16 unterbrochen. Alternativ oder zusätzlich ist es möglich, die elektrischen Parameter an der Ausgangsseite des Transformators 26 zu überwachen. In diesem Fall weist beispielsweise der erste Anschluss 28a der Ausgangsseite 28 eine zusätzliche Verbindung zur Sicherheitseinrichtung 20 auf.

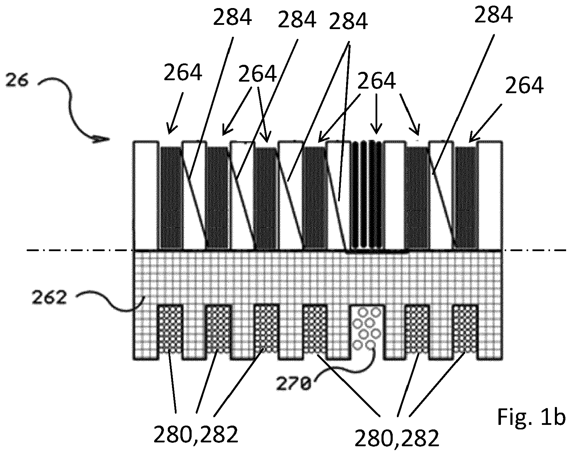

Figur 1b zeigt eine schematische Schnittansicht des als Resonanztransformator ausgestalteten Transformators 26. Die Ansicht ist zweigeteilt, wobei die obere Hälfte eine Draufsicht und die untere Hälfte eine Schnittansicht des Transformators 26 zeigt. Der Transformator 26 weist einen Kern 262 auf, welcher in dem in der Figur 1b dargestellten Beispiel sieben Spulenaufnahmen 264 aufweist.

Die Sekundärspule 280 des Transformators 26 ist in dem dargestellten Beispiel in sechs Spulenabschnitte 282 aufgeteilt, wobei jeweils einer der Spulenabschnitte 282 in einer der Spulenaufnahmen 264 angeordnet ist. Die einzelnen Spulenabschnitte 282 sind durch die Spulenaufnahmen 264 voneinander beabstandet angeordnet. Elektrisch sind die einzelnen Spulenabschnitte 282 in Reihe geschaltet, wobei jeweils zwei benachbart angeordnete Spulenabschnitte 282 mit einer Verbindung 284 miteinander elektrisch verbunden sind. Durch den Abstand zwischen jeweils zweien der Spulenabschnitte 282 zueinander bildet sich jeweils eine elektrische Kapazität aus, welche die Eigenfrequenz bzw. Resonanzfrequenz des schwingfähigen Systems auf der Ausgangsseite 28, vergleiche Figur 1a, des Transformators 26 beeinflusst. Entsprechend kann diese Resonanzfrequenz durch Ändern der Abstände eingestellt werden.

Die Primärspule 270 ist in dem dargestellten Beispiel als ein einziger Abschnitt ausgestaltet, der ebenfalls in einer der Spulenaufnahmen 264 angeordnet ist. Dabei befindet sich die Primärspule 270 zwischen zwei Spulenabschnitten 282 der Sekundärspule 280.

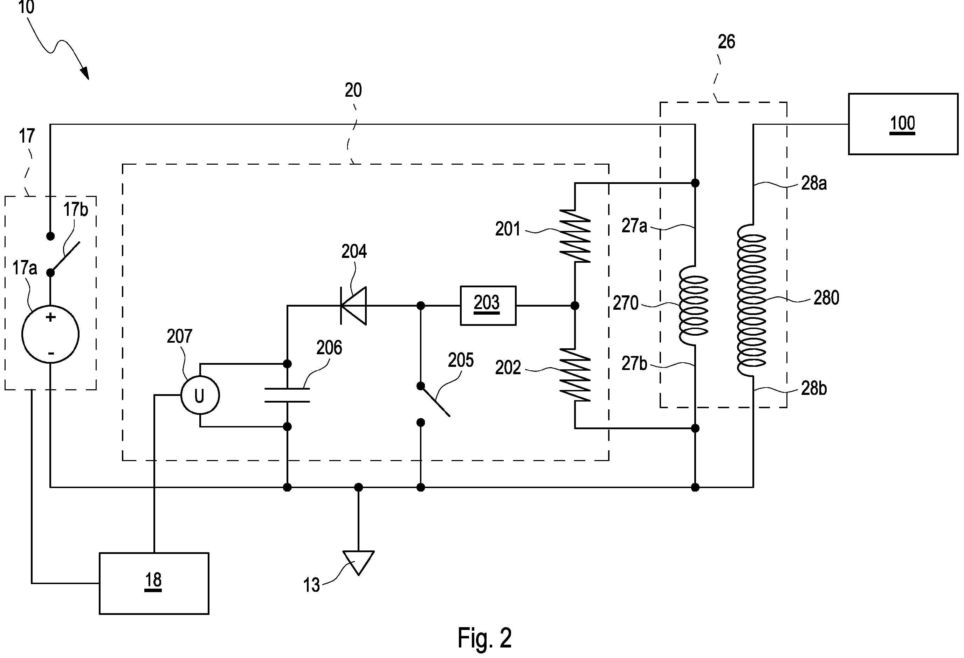

Figur 2 zeigt ein vereinfachtes schematisches Schaltbild der Vorrichtung 10. Dabei ist insbesondere dargestellt, wie die Sicherheitseinrichtung 20 zur Messung elektrischer Parameter mit dem Transformator 26 verbunden ist. Der Transformator 26 ist als impulsbetriebener Transformator ausgeführt und weist eine Primärspule 270 und eine Sekundärspule 280 auf. Ein erster Anschluss 27a der Eingangsseite 27 bzw. der Primärspule 270 ist mit dem Pulsgenerator 17 verbunden. Ein zweiter Anschluss 27b der Eingangsseite 27 bzw. der Primärspule 270 ist mit der Gehäuseabschirmung 13 verbunden. Ein erster Anschluss 28a der Ausgangsseite 28 bzw. der Sekundärspule 280 ist mit dem Behandlungsinstrument 100 verbunden und ein zweiter Anschluss 28b der Ausgangsseite 28 bzw. der Sekundärspule 280 ist ebenfalls mit der Gehäuseabschirmung 13 verbunden.

Der Pulsgenerator 17 umfasst in der vereinfachten Darstellung der Figur 2 eine Gleichspannungsquelle 17a, welche für die Dauer eines elektrischen Anregungspulses über den Schalter 17b mit dem Transformator 26 verbunden wird.

Die schematisch dargestellte Sicherheitseinrichtung 20 ist in der Ausführungsform der Figur 2 dazu eingerichtet, elektrische Parameter der hochfrequenten elektrischen Pulse über eine Messung der von diesen hochfrequenten elektrischen Pulsen in der Primärspule induzierten Spannung zu ermitteln. Hierzu ist die Sicherheitseinrichtung 20 mit den ersten und zweiten Anschlüssen 27a, 27b der Primärspule 270 verbunden.

Die Sicherheitseinrichtung 20 kontrolliert die elektrischen Parameter der hochfrequenten elektrischen Pulse über das Messen einer Spannung an einem Kondensator 206, welcher durch einen in der Primärspule 270 induzierten Strom aufgeladen wird. Hierzu ist der Kondensator 207 über eine Diode 204 und einen Impedanzwandler 203 und einen durch einen ersten Widerstand 201 und einen zweiten Widerstand 202 gebildeten Spannungsteiler mit dem ersten Anschluss 27a der Primärspule 270 verbunden. Über einen steuerbaren Schalter 205, der beispielsweise als MOSFET ausgestaltet ist, kann während des elektrischen Anregungspulses der Ausgang des Impedanzwandlers 203 mit der Gehäuseabschirmung 13 und somit mit dem zweiten Anschluss 27b der Primärspule 270 kurzgeschlossen werden, so dass der elektrische Anregungspuls den Kondensator 206 nicht aufladen kann. Erst nach Beenden der Anregung des Transformators 26 wird der steuerbare Schalter 205 sperrend geschaltet, so dass jeweils die positiven Halbwellen der im Transformator 26 vorliegenden Schwingung den Kondensator 206 aufladen. Die am Kondensator 206 anliegende Spannung kann dann mit Spannungsmessmitteln 207 gemessen werden. Die Steuerung 18 kann dann abhängig vom Ergebnis der Spannungsmessung den Pulsgenerator 17 steuern und z.B. bei Über- oder Unterschreitung vorgegebener Grenzwerte den Pulsgenerator 17 abschalten.

In der Darstellung der Figur 2 sind der Pulsgenerator 17, die Sicherheitseinrichtung 20 und die Steuerung 18 als separate Komponenten dargestellt. Es kann jedoch vorgesehen sein, mehrere oder alle diese Komponenten als eine gemeinsame Einheit auszugestalten, wie dies in Figur 1a angedeutet ist.

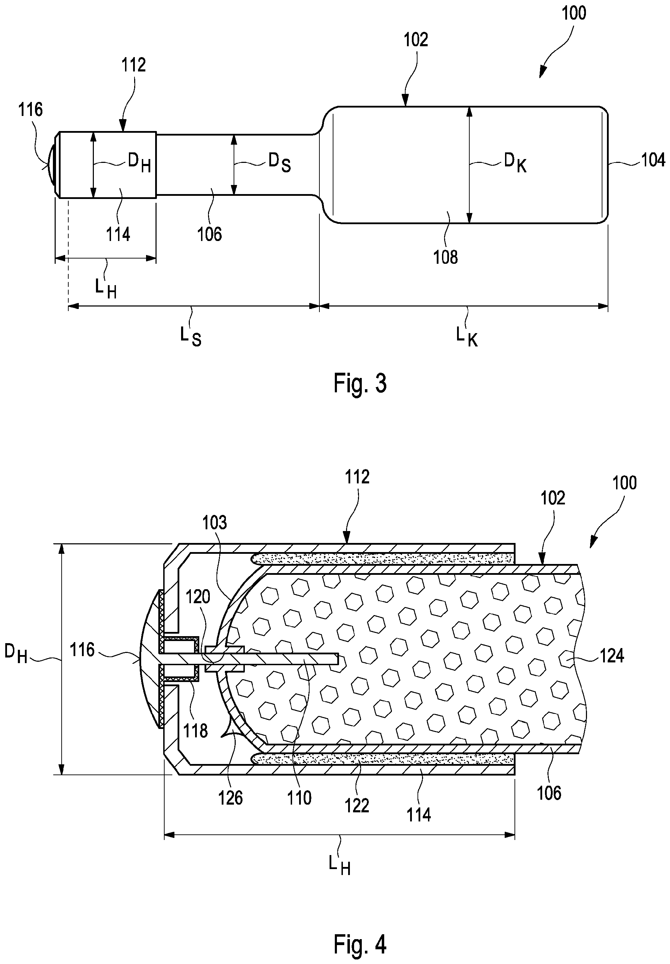

In Figur 3 ist das Behandlungsinstrument 100 der Vorrichtung 10 schematisch dargestellt.

Das Behandlungsinstrument 100 umfasst einen Körper 102, welcher aus einem elektrisch isolierenden Material wie beispielsweise Glas gefertigt ist.

An das erste Ende 103 des Behandlungsinstrument 100 grenzt der Stegbereich 106 an, welcher einen ersten Durchmesser Ds aufweist, und an das zweite Ende 104 des Behandlungsinstruments 100 grenzt der Kolbenbereich 108 an, der einen zweiten Durchmesser D_{K} aufweist. Der zweite Durchmesser D_{K} ist dabei größer als der erste Durchmesser Ds. Der Stegbereich 106 weist eine erste Länge Ls und der Kolbenbereich 108 weist eine zweite Länge L_{K} auf. Die erste Länge Ls des Stegbereichs 106 nimmt in der dargestellten Ausführungsform etwa 45% der Gesamtlänge des Körpers 102 ein.

Bevorzugt nimmt das Volumen des Kolbenbereichs 108 mindestens zwei Drittel des Gesamtvolumens des Körpers 102 des Behandlungsinstruments 100 ein. Wie der Darstellung der Figur 3 entnommen werden kann, gehen der Kolbenbereich 108 und der Stegbereich 106 nicht sprunghaft ineinander über. Der Körper 102 weist einen kontinuierlichen Übergang zwischen dem Stegbereich 106 und dem Kolbenbereich 108 auf.

Das zweite Ende 104 des Körpers 102 des Behandlungsinstruments 100 ist in der dargestellten Ausführungsform im Wesentlichen plan ausgestaltet, so dass eine kreisrunde Fläche den Kolbenbereich 108 abschließt. Diese Fläche geht über einen kontinuierlichen Übergang in den kreiszylinderförmigen Kolbenbereich 108 über. Für einen Kontakt mit einem zu behandelnden Objekt bzw. mit zu behandelndem organischem Material kann sowohl die im Wesentlichen plane Fläche als auch die gebogene Wandung des Kolbenbereichs 108 verwendet werden.

Die Abschlusskappe 112 umschließt den Körper 102 des Behandlungsinstruments 100 am ersten Ende 103, wobei eine elektrische Kontaktfläche 116, vergleiche Figuren 1 und 3, am ersten Ende 103 frei bleibt. Die Abschlusskappe 112 umfasst einen Hülsenbereich 114, welcher sich über zumindest einen Teil des Stegbereichs 106 ausgehend vom ersten Ende erstreckt. Der Hülsenbereich 114 weist eine Länge L_{H} und einen Durchmesser D_{H} auf.

Figur 4 zeigt eine Detailansicht des Behandlungsinstruments 100 welche den an das erste Ende 103 angrenzenden Teil in einer Schnittansicht zeigt. Zu erkennen ist, dass die Abschlusskappe 112 den Körper 102 des Behandlungsinstruments 100 am ersten Ende 103 umschließt, wobei eine elektrische Kontaktfläche 116 durch die Abschlusskappe 112 hindurch die Elektrode 110 im Inneren des Körpers 112 kontaktiert. Zwischen der elektrischen Kontaktfläche 116 und einer elektrischen Durchführung 120 durch den Körper 102 hindurch ist in der in Figur 3 gezeigten Ausführungsform eine Niete 118 aus einem elektrisch isolierenden Material angeordnet, welche eine elektrische Verbindung zwischen der elektrischen Durchführung 120 und der elektrischen Kontaktfläche 116 umgibt.

Des Weiteren ist in der Figur 4 zu erkennen, dass die Abschlusskappe 112 eine Evakuierungsöffnung 126 des Körpers 102 abdeckt. Über die Evakuierungsöffnung 126 wurde bei der Herstellung des Körpers 102 zunächst Luft evakuiert und anschließend ein Edelgas 124, wie beispielsweise Neon, in das Innere des Körpers 102 eingeleitet. Danach wurde die Evakuierungsöffnung 126 verschlossen.

Die Abschlusskappe 112 umfasst des Weiteren den Hülsenbereich 114, welcher sich über zumindest einen Teil des Stegbereichs 106 ausgehend vom ersten Ende 103 erstreckt. Der Hülsenbereich 114 weist eine Länge L_{H} und einen Durchmesser D_{H} auf. Zum Befestigen der Abschlusskappe 112 an dem Körper 102 wird in der in Figur 3 dargestellten Ausführungsform ein Epoxidkleber 122 verwendet, welcher zwischen dem Körper 102 und dem Hülsenbereich 114 der Abschlusskappe 112 angeordnet ist.

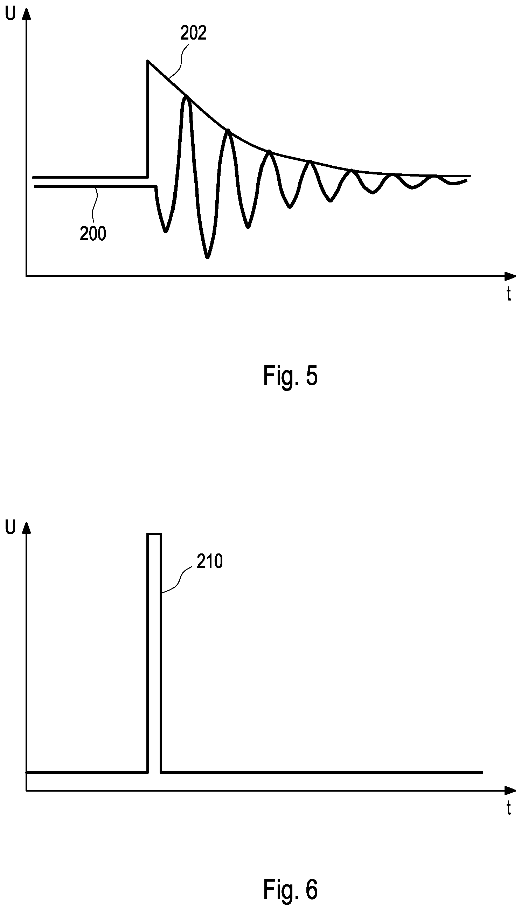

Figur 5 zeigt den qualitativen Verlauf eines hochfrequenten elektrischen Pulses 200 an der Ausgangsseite 27 des Transformators 26. Der hochfrequente elektrische Puls 200 entsteht nach Beaufschlagung des Transformators 26 mit einem Anregungspuls 210, der in der Figur 6 dargestellt ist und der von dem Pulsgenerator 17 der Steuerung 18 erzeugt wird. Der Anregungspuls 210 regt den als Resonanztransformator ausgestalteten Transformator 26, vergleiche Figur 1a, zu einer Schwingung an, so dass ein hochfrequentes Signal mit hoher Spannung erzeugt wird, welches als hochfrequenter elektrischer Puls 200 zur elektrischen Anregung des Behandlungsinstruments 100 verwendet wird.

In den Figuren 5 und 6 ist auf der Y-Achse die Spannung und auf der X-Achse die Zeit aufgetragen. Der in Figur 6 gezeigte Anregungspuls 210 wird beispielsweise erhalten, indem über einen Halbleiterschalter, beispielsweise ein MOSFET, eine Gleichspannung ein und wieder ausgeschaltet wird. Die Figuren 5 und 6 zeigen jeweils einen einzelnen Puls.

Der in Figur 5 dargestellte hochfrequente elektrische Puls 200 weist eine Einhüllende 202 auf, welche in dem gezeigten Beispiel einem exponentiellen Abfall folgt. Eine Pulsbreite bzw. Pulsdauer des hochfrequenten elektrischen Pulses 200 kann hier als die Zeit definiert werden, die bis zum Abfall der Einhüllenden 202 auf einen Wert von A/e vergeht, wobei A die höchste Amplitude ist und e die Eulersche Zahl ist.

Die Erfindung ist nicht auf die hier beschriebenen Ausführungsbeispiele und die darin hervorgehobenen Aspekte beschränkt. Vielmehr ist innerhalb des durch die Ansprüche angegebenen Bereichs eine Vielzahl von Abwandlungen möglich, die im Rahmen fachmännischen Handelns liegen.

### Bezugszeichenliste

- 10: Vorrichtung
- 12: Gehäuse
- 12a: Griffabschnitt
- 13: Gehäuseabschirmung
- 14: Instrumentenführung
- 16: elektrischer Energiespeicher
- 17: Pulsgenerator
- 17a: Spannungsquelle
- 17b: Schalter
- 18: Steuerung
- 20: Sicherheitseinrichtung
- 201: erster Widerstand
- 202: zweiter Widerstand
- 203: Impedanzwandler
- 204: Diode
- 205: Schalter
- 206: Kondensator
- 207: Spannungsmessmittel
- 22: Ladeanschluss
- 24: Bedienelement
- 26: Transformator
- 262: Kern
- 264: Spulenaufnahme
- 27: Eingangsseite
- 27a: erster Anschluss Eingangsseite
- 27b: zweiter Anschluss Eingangsseite
- 270: Primärspule
- 28: Ausgangsseite
- 28a: erster Anschluss Ausgangsseite
- 28b: zweiter Anschluss Ausgangsseite
- 280: Sekundärspule
- 282: Spulenabschnitt
- 284: Verbindung
- 30: elektrischer Federkontakt
- 32: Klemmeinrichtung
- 100: Behandlungsinstrument
- 102: Körper
- 103: erste Seite
- 104: zweite Seite
- 106: Stegbereich
- 108: Kolbenbereich
- 110: Elektrode
- 112: Abschlusskappe
- 114: Hülsenbereich
- 116: elektrische Kontaktfläche
- 118: Niete
- 120: elektrische Durchführung
- 122: Epoxidkleber
- 124: Edelgas
- 126: Evakuierungsöffnung
- 200: hochfrequenter elektrischer Puls
- 202: Einhüllende
- 210: Anregungspuls
- D: Durchmesser (S=Stegbereich, H=Hülsenbereich, K=Kolbenbereich)
- L: Länge (S=Stegbereich, H=Hülsenbereich, K=Kolbenbereich)

## Patentansprüche

1. Vorrichtung (10) zur Unterstützung einer Behandlung mit gepulsten elektrischen Feldern zur Wundheilung und/oder zur Inaktivierung von Mikroorganismen umfassend
- einen elektrischen Energiespeicher (16),
- einen Pulsgenerator (17) zur Bereitstellung elektrischer Anregungspulse,
- einen Transformator (26) zum Bereitstellen hochfrequenter elektrischer Pulse (200) an einer Ausgangsseite (28), wobei ein erster Anschluss einer Eingangsseite (27) des Transformators (26) mit dem Pulsgenerator (17) verbunden ist und
- ein Behandlungsinstrument (100), welches einen Körper (102) aus einem elektrisch isolierenden Material und eine im Inneren des Körpers (102) angeordnete Elektrode (110) umfasst, wobei im Inneren des Körpers (102) ein Gas oder Gasgemisch aufgenommen ist und das Behandlungsinstrument (100) für eine Gasentladung bei elektrischer Anregung eingerichtet ist, wobei ein erstes Ende (103) des Behandlungsinstruments (100) für eine Kopplung mit einem ersten Anschluss der Ausgangsseite (28) des Transformators (26) eingerichtet ist, wobei ein zweites Ende (104) des Behandlungsinstruments (100) zum Kontaktieren von Oberflächen und/oder von biologischem Gewebe eingerichtet ist und dass ein zweiter Anschluss der Ausgangsseite (28) des Transformators (26) mit einem zweiten Anschluss einer Eingangsseite (27) des Transformators (26) verbunden ist, wobei der Transformator (26) und der Pulsgenerator (17) derart eingerichtet sind, dass die hochfrequenten elektrischen Pulse (200)
- eine Pulswiederholrate im Bereich von 100 Hz bis 400 Hz aufweisen, wobei
das Behandlungsinstrument (100) dazu eingerichtet ist, die erzeugten hochfrequenten elektrischen Pulse (200) bzw. deren elektrische Felder zu einem zu behandelnden Objekt oder zu einem zu behandelnden organischen bzw. biologischen Material zu leiten, **dadurch gekennzeichnet, dass** besagter Körper (102) ein geschlossenen Körper (102) ist, wobei die Vorrichtung ein Gehäuse, sowie eine Gehäuseabschirmung (13) aufweist mit welcher besagter zweiter Anschluss der Ausgangsseite (28) des Transformators (26) verbunden ist, wobei die Gehäuseabschirmung (13) durch ein Gehäuse der Vorrichtung (10) elektrisch von der Außenwelt isoliert ist, und somit als erdfreie Masse eingerichtet ist, wobei der Pulsgenerator (17) derart eingerichtet ist, dass die hochfrequenten elektrischen Pulse (200) eine Frequenz im Bereich von 10 kHz bis 100 kHz aufweisen.

2. Vorrichtung (10) nach Anspruch 1, **dadurch gekennzeichnet, dass** die Vorrichtung (10) des Weiteren eine Sicherheitseinrichtung (20) umfasst, welche eingerichtet ist, eine Spannung und/oder eine Frequenz der hochfrequenten elektrischen Pulse (200) an der Ausgangsseite (28) des Transformators (26) zu messen und eine Energiezufuhr zum Transformator (26) zu unterbrechen, wenn eine Spannung und/oder eine Frequenz der hochfrequenten elektrischen Pulse (200) vorgegebene Grenzwerte über und/oder unterschreiten und/oder
welche eingerichtet ist, eine elektrische Verbindung der Vorrichtung (10) zu einem Stromnetz zu erkennen und bei Vorliegen einer solchen Verbindung eine Energiezufuhr zum Transformator (26) zu unterbrechen.

3. Vorrichtung (10) nach Anspruch 1 oder 2, **dadurch gekennzeichnet, dass** der Transformator (26) als ein impulsbetriebener Transformator ausgebildet ist und für das Bereitstellen der hochfrequenten elektrischen Pulse (200) mit Anregungspulsen (210) angeregt wird, wobei eine Wiederholrate der Anregungspulse (210) der Pulswiederholrate der hochfrequenten elektrischen Pulse (200) entspricht.

4. Vorrichtung (10) nach Anspruch 3, weiters konfiguriert, dass eine Anregung des impulsbetriebenen Transformators durch Verschieben der Lage der Primärspule des Transformators in Bezug auf die Sekundärspule des Transformators einstellbar ist.

5. Vorrichtung (10) nach Anspruch 2 und Anspruch 3 oder 4, **dadurch gekennzeichnet, dass** die Messung der Spannung und/oder Frequenz der hochfrequenten elektrischen Pulse (200) während einer Anregungspause des impulsbetriebenen Transformators über eine Messung der an der Primärseite des impulsbetriebenen Transformators induzierten Spannung erfolgt.

6. Vorrichtung (10) nach einem der Ansprüche 1 bis 5, **dadurch gekennzeichnet, dass** das Behandlungsinstrument (100) am ersten Ende (103) eine Stromdurchführung für die Elektrode (110) aufweist und die Stromdurchführung eine Abschlusskappe (112) aus einem Kunststoff umfasst.

7. Vorrichtung (10) nach Anspruch 6, **dadurch gekennzeichnet, dass** der Kunststoff der Abschlusskappe (112) mit Fasern verstärkt ist.

8. Vorrichtung (10) nach einem der Ansprüche 1 bis 7, **dadurch gekennzeichnet, dass** der Körper (102) des Behandlungsinstruments (100) mit einem Edelgas (124) oder einem Edelgasgemisch mit einem Druck im Bereich von 0,001 mbar bis 7 mbar gefüllt ist.

9. Vorrichtung (10) nach einem der Ansprüche 1 bis 8, **dadurch gekennzeichnet, dass** an das erste Ende (103) des Behandlungsinstruments (100) ein Stegbereich (106) mit einem ersten Durchmesser Ds angrenzt und an das zweite Ende (104) des Behandlungsinstruments (100) ein Kolbenbereich (108) mit einem zweiten Durchmesser D_{K} angrenzt, wobei der zweite Durchmesser D_{K} größer ist als der erste Durchmesser Ds und wobei der Kolbenbereich (108) mindestens ein Drittel der Gesamtlänge des Körpers (102) einnimmt.

10. Vorrichtung (10) nach Anspruch 6 oder 7 und Anspruch 9, **dadurch gekennzeichnet, dass** die Abschlusskappe (112) einen Hülsenbereich (114) aufweist, welcher den Stegbereich (106) des Behandlungsinstruments (100) zumindest teilweise umschließt, und dass das Behandlungsinstrument (100) über eine Klemmeinrichtung (32) am Hülsenbereich (114) gehalten wird.

11. Vorrichtung (10) nach einem der Ansprüche 1 bis 10, **dadurch gekennzeichnet, dass** das Behandlungsinstrument (100) für eine Autoklavierung mit Wasserdampf bei einer Temperatur im Bereich von 110 °C bis 140 °C eingerichtet ist.

12. Verfahren zur Inaktivierung von Mikroorganismen, wobei eine Vorrichtung (10) nach einem der Ansprüche 1 bis 11 bereitgestellt wird und das Behandlungsinstrument (100) der Vorrichtung (10) mit hochfrequenten elektrischen Pulsen (200) angeregt wird, **dadurch gekennzeichnet, dass** das Behandlungsinstrument (100) auf eine Oberfläche eines Objekts, bei dem Mikroorganismen inaktiviert werden sollen, aufgesetzt wird, so dass der Körper (102) des Behandlungsinstruments (100) die Oberfläche berührt, und das Behandlungsinstrument (100) über die Oberfläche des Objekts geführt wird, wobei die Gehäuseabschirmung (13) elektrisch von dem Objekt getrennt ist, so dass keine elektrisch leitende Verbindung zwischen dem Objekt und den zweiten Anschlüssen (27b, 28b) des Transformators (26) hergestellt wird, wobei das Objekt ausgewählt ist aus medizinischen Instrumenten und organischem Material, wobei das organische Material eine Gewebeprobe ist.

13. Verfahren nach Anspruch 12, **dadurch gekennzeichnet, dass** auch im Inneren des Objekts Mikroorganismen inaktiviert werden

## Claims

1. Device (10) for supporting a treatment with pulsed electric fields for wound healing and/or for inactivating microorganisms, comprising
- an electrical energy storage (16),
- a pulse generator (17) for providing electrical excitation pulses,
- a transformer (26) for providing high-frequency electrical pulses (200) at an output side (28), wherein a first terminal of an input side (27) of the transformer (26) is connected to the pulse generator (17), and
- a treatment instrument (100) comprising a body (102) made of an electrically insulating material and an electrode (110) arranged inside the body (102), wherein a gas or gas mixture is accommodated inside the body (102) and the treatment instrument (100) is configured for gas discharge upon electrical excitation, wherein a first end (103) of the treatment instrument (100) is configured for coupling to a first terminal of the output side (28) of the transformer (26), wherein
a second end (104) of the treatment instrument (100) is configured to contact surfaces and/or biological tissue, and that a second terminal of the output side (28) of the transformer (26) is connected to a second terminal of an input side (27) of the transformer (26), wherein the transformer (26) and the pulse generator (17) are configured in such a way that the high-frequency electrical pulses (200) have a pulse repetition rate in the range from 100 Hz to 400 Hz, wherein the treatment instrument (100) is configured to conduct the generated high-frequency electric pulses (200) or their electric fields to an object to be treated or to an organic or biological material to be treated,
**characterized in that** said body (102) is a closed body (102), wherein the device comprises a housing and a housing shield (13) to which said second terminal of the output side (28) of the transformer (26) is connected, wherein the housing shield (13) is electrically insulated from the outside world by a housing of the device (10) and thus is configured as a floating ground, wherein the pulse generator (17) is configured in such a way that the high-frequency electrical pulses (200) have a frequency in the range from 10 kHz to 100 kHz.

2. Device (10) according to claim 1, **characterized in that** the device (10) further comprises a safety device (20) which is configured to measure a voltage and/or a frequency of the high-frequency electrical pulses (200) at the output side (28) of the transformer (26) and to interrupt a power supply to the transformer (26), if a voltage and/or a frequency of the high-frequency electrical pulses (200) exceed and/or fall below predetermined limit values and/or which is configured to recognize an electrical connection of the device (10) to a power supply system and to interrupt a power supply to the transformer (26) if such a connection is present.

3. Device (10) according to claim 1 or 2, **characterized in that** the transformer (26) is designed as a pulse-driven transformer and is excited with excitation pulses (210) for providing the high-frequency electrical pulses (200), wherein a repetition rate of the excitation pulses (210) corresponds to the pulse repetition rate of the high-frequency electrical pulses (200).

4. Device (10) according to claim 3, further configured in that an excitation of the pulse-operated transformer is adjustable by shifting the position of the primary coil of the transformer with respect to the secondary coil of the transformer.

5. Device (10) according to claim 2 and claim 3 or 4, **characterized in that** the measurement of the voltage and/or frequency of the high-frequency electrical pulses (200) during an excitation pause of the pulse-driven transformer is carried out via a measurement of the voltage induced at the primary side of the pulse-driven transformer.

6. Device (10) according to one of claims 1 to 5, **characterized in that** the treatment instrument (100) has a current feedthrough for the electrode (110) at the first end (103) and the current feedthrough comprises an end cap (112) made of a plastic.

7. Device (10) according to claim 6, **characterized in that** the plastic of the end cap (112) is reinforced with fibres.

8. Device (10) according to one of claims 1 to 7, **characterized in that** the body (102) of the treatment instrument (100) is filled with a noble gas (124) or a noble gas mixture with a pressure in the range of 0.001 mbar to 7 mbar.

9. Device (10) according to one of claims 1 to 8, **characterized in that** a bar region (106) with a first diameter Dₛ adjoins the first end (103) of the treatment instrument (100) and a piston region (108) with a second diameter D_{K} adjoins the second end (104) of the treatment instrument (100), wherein the second diameter D_{K} is larger than the first diameter Dₛ and wherein the piston region (108) takes up at least one third of the total length of the body (102).

10. Device (10) according to claim 6 or 7 and claim 9, **characterized in that** the end cap (112) has a sleeve region (114) which at least partially encloses the bar region (106) of the treatment instrument (100), and **in that** the treatment instrument (100) is held on the sleeve region (114) via a clamping device (32).

11. Device (10) according to one of claims 1 to 10, **characterized in that** the treatment instrument (100) is configured for autoclaving with steam at a temperature in the range from 110 °C to 140 °C.

12. Method for inactivating microorganisms, wherein a device (10) according to one of claims 1 to 11 is provided and the treatment instrument (100) of the device (10) is excited with high-frequency electrical pulses (200), **characterized in that** the treatment instrument (100) is placed on a surface of an object in which microorganisms are to be inactivated, so that the body (102) of the treatment instrument (100) touches the surface, and the treatment instrument (100) is guided over the surface of the object, wherein the housing shield (13) is electrically separated from the object so that no electrically conductive connection is established between the object and the second terminals (27b, 28b) of the transformer (26), wherein the object is selected from medical instruments and organic material, wherein the organic material is a tissue sample.

13. Method according to claim 12, **characterized in that** microorganisms are also inactivated inside the object.

## Revendications

1. Dispositif (10) permettant d'assister un traitement par champs électriques pulsés destiné à la guérison de plaies et/ou à l'inactivation de microorganismes, comprenant
- un accumulateur d'énergie électrique (16),
- un générateur d'impulsions (17) pour fournir des impulsions d'excitation électriques,
- un transformateur (26) pour fournir des impulsions électriques haute fréquence (200) sur un côté sortie (28), dans lequel une première borne d'un côté entrée (27) du transformateur (26) est reliée au générateur d'impulsions (17), et
- un instrument de traitement (100) qui comprend un corps (102) d'un matériau électriquement isolant et une électrode (110) disposée à l'intérieur du corps (102), dans lequel un gaz ou mélange gazeux est reçu à l'intérieur du corps (102), et l'instrument de traitement (100) est conçu pour une décharge de gaz en cas d'excitation électrique, dans lequel une première extrémité (103) de l'instrument de traitement (100) est conçue pour un couplage à une première borne du côté sortie (28) du transformateur (26), dans lequel une deuxième extrémité (104) de l'instrument de traitement (100) est conçue pour entrer en contact avec des surfaces et/ou un tissu biologique, et
une deuxième borne du côté sortie (28) du transformateur (26) qui est reliée à une deuxième borne d'un côté entrée (27) du transformateur (26), dans lequel le transformateur (26) et le générateur d'impulsions (17) sont conçus de telle sorte que les impulsions électriques haute fréquence (200)
- présentent une fréquence de répétition d'impulsion dans la plage de 100 Hz à 400 Hz, dans lequel
l'instrument de traitement (100) est conçu pour conduire les impulsions électriques haute fréquence (200) générées ou leurs champs électriques jusqu'à un objet à traiter ou jusqu'à une matière organique ou biologique à traiter,
**caractérisé en ce que** ledit corps (102) est un corps fermé (102), dans lequel le dispositif présente un boîtier, ainsi qu'un blindage de boîtier (13) auquel est relié ladite deuxième borne du côté sortie (28) du transformateur (26), dans lequel le blindage de boîtier (13) est électriquement isolé par rapport à l'extérieur par un boîtier du dispositif (10) et est donc conçu sous forme de masse isolée de la terre, dans lequel le générateur d'impulsions (17) est conçu de telle sorte que les impulsions électriques haute fréquence (200) présentent une fréquence dans la plage de 10 kHz à 100 kHz.

2. Dispositif (10) selon la revendication 1, **caractérisé en ce que** le dispositif (10) comprend de plus un équipement de sécurité (20) qui est conçu pour mesurer une tension et/ou une fréquence des impulsions électriques haute fréquence (200) sur le côté sortie (28) du transformateur (26) et pour couper une alimentation en énergie vers le transformateur (26) lorsqu'une tension et/ou une fréquence des impulsions électriques haute fréquence (200) dépassent et/ou soupassent des valeurs seuils prédéfinies, et/ou qui est conçu pour identifier une liaison électrique du dispositif (10) à un réseau électrique et pour couper une alimentation en énergie vers le transformateur (26) si une telle liaison existe.

3. Dispositif (10) selon la revendication 1 ou 2, **caractérisé en ce que** le transformateur (26) est réalisé sous la forme d'un transformateur actionné par impulsions et est excité pour fournir des impulsions électriques haute fréquence (200) avec des impulsions d'excitation (210), dans lequel une fréquence de répétition des impulsions d'excitation (210) correspond à la fréquence de répétition d'impulsion des impulsions électriques haute fréquence (200).

4. Dispositif (10) selon la revendication 3, configuré en outre de telle sorte qu'une excitation du transformateur actionné par impulsions peut être réglée par le décalage de la position de la bobine primaire du transformateur par rapport à la bobine secondaire du transformateur.

5. Dispositif (10) selon la revendication 2 et la revendication 3 ou 4, **caractérisé en ce que** la mesure de la tension et/ou de la fréquence des impulsions électriques haute fréquence (200) a lieu pendant une pause d'excitation du transformateur actionné par impulsions par une mesure de la tension induite sur le côté primaire du transformateur actionné par impulsions.

6. Dispositif (10) selon l'une quelconque des revendications 1 à 5, **caractérisé en ce que** l'instrument de traitement (100) présente à la première extrémité (103) un passage de courant pour l'électrode (110), et le passage de courant comprend un capuchon d'extrémité (112) en matière plastique.

7. Dispositif (10) selon la revendication 6, **caractérisé en ce que** la matière plastique du capuchon d'extrémité (112) est renforcée par des fibres.

8. Dispositif (10) selon l'une quelconque des revendications 1 à 7, **caractérisé en ce que** le corps (102) de l'instrument de traitement (100) est rempli d'un gaz noble (124) ou d'un mélange de gaz nobles à une pression dans la plage de 0,001 mbar à 7 mbar.

9. Dispositif (10) selon l'une quelconque des revendications 1 à 8, **caractérisé en ce qu'**une zone de tige (106) d'un premier diamètre Ds est adjacente à la première extrémité (103) de l'instrument de traitement (100), et une zone de piston (108) d'un deuxième diamètre D_{K} est adjacente à la deuxième extrémité (104) de l'instrument de traitement (100), dans lequel le deuxième diamètre D_{K} est supérieur au premier diamètre Ds, et dans lequel la zone de piston (108) occupe au moins un tiers de la longueur totale du corps (102).

10. Dispositif (10) selon la revendication 6 ou 7 et la revendication 9, **caractérisé en ce que** le capuchon d'extrémité (112) présente une zone de manchon (114) qui entoure au moins en partie la zone de tige (106) de l'instrument de traitement (100), et **en ce que** l'instrument de traitement (100) est maintenu par un équipement de serrage (32) au niveau de la zone de manchon (114).

11. Dispositif (10) selon l'une quelconque des revendications 1 à 10, **caractérisé en ce que** l'instrument de traitement (100) est conçu pour un autoclavage à la vapeur d'eau à une température dans la plage de 110 °C à 140 °C.

12. Procédé d'inactivation de microorganismes, dans lequel un dispositif (10) selon l'une quelconque des revendications 1 à 11 est fourni, et l'instrument de traitement (100) du dispositif (10) est excité par des impulsions électriques haute fréquence (200),
**caractérisé en ce que** l'instrument de traitement (100) est posé sur une surface d'un objet pour lequel des microorganismes sont à inactiver de sorte que le corps (102) de l'instrument de traitement (100) touche la surface, et l'instrument de mesure (100) est guidé sur la surface de l'objet, dans lequel le blindage de boîtier (13) est électriquement isolé de l'objet de sorte qu'aucune liaison électriquement conductrice ne soit établie entre l'objet et les deuxièmes bornes (27b, 28b) du transformateur (26), dans lequel l'objet est sélectionné parmi des instruments médicaux ou des matières organiques, dans lequel la matière organique est un échantillon de tissu.

13. Procédé selon la revendication 12, **caractérisé en ce que** des microorganismes sont inactivés même à l'intérieur de l'objet.
